# EUROPEAN PATENT SPECIFICATION

(11) **EP 0 414 806 B1**
(45) Date of publication and mention of the grant of the patent: **20.07.1994**
(21) Application number: 89906648.4
(22) Date of filing: 22.05.1989
(51) Int. Cl.: A61K 9/50, A61K 9/52, B01J 13/02

(54) **HIGH RATIO ACTIVE AGENT:LIPID COMPLEX**
AKTIVES MITTEL: LIPID-KOMPLEX MIT HOHEM VERHÄLTNIS
COMPLEXE AGENT ACTIF:LIPIDE A RAPPORT ELEVE

(30) Priority: 20.05.1988 US 196913
(43) Date of publication of application: 06.03.1991
(73) Proprietor: THE LIPOSOME COMPANY, INC., Princeton, NJ 08540 (US)
(72) Inventor: MINCHEY, Sharma, R., Monmouth Junction, NJ 08852 (US); SWENSON, Christine, E., Princeton Junction, NJ 08550 (US); JANOFF, Andrew, S., Yardley, PA 19087 (US); BONI, Lawrence, Highland Park, NJ 08904 (US); STEWART, Kathy, A., Morrison, IL 61270 (US); PERKINS, Walter, Plainsboro, NJ 08536 (US)
(74) Representative: Ahner, Francis
(86) International application number: US8902224
(87) International publication number: WO8911272

(56) References cited:
- WO-A-85/00515
- US-A- 4 137 317
- US-A- 4 192 859
- US-A- 4 235 871
- US-A- 4 438 052
- US-A- 4 522 803
- US-A- 4 544 545
- US-A- 4 567 034
- US-A- 4 588 578
- US-A- 4 673 567
- US-A- 4 708 861
- US-A- 4 735 795
- US-A- 4 753 788
- US-A- 4 830 858
- THE JOURNAL OF INFECTIOUS DISEASES, VOL. 147, NO. 5, MAY 1983 (83.5) (THE UNIVERSITY OF CHICAGO, USA), G. LOPEZ-BERESTEIN ET AL.: "TREATMENT AND PROPHYLAXIS OF DISSEMINATED INFECTION DUE TO CANDIDA ALBICANS IN MICE WITH LIPOSOME-ENCAPSULATED AMPHOTERICIN B2
- CHEMICAL ABSTRACTS, VOL. 87, NO. II, ISSUED 1977 (COLOMBUS, OHIO, USA), M. YASUHARA ET AL.: "INTESTINAL ABSORPTION ASPECTS OF NON-LIPOPLILIC LOW MOLECULAR WEIGHT DRUGS: CASE OF CEPHALEXIN AND CEPHAZOLIN" , ABSTRACT NO. 78182B, CHEM. PHARM. BULLETIN 25(4), 675-679, 1977
- CHEMICAL ABSTRACTS, VOL. 99, NO. 26, ISSUED 1983 (COLOMBUS, OHIO), J.V. DESIDERO ET AL.: "LIPOSOME-ENCAPSULATED CEPHALOTHIN IN THE TREATMENT OF EXPERIMENTAL MURINE SALMONELLOSIS", SEE ABSTRACT NO. 218501J, J. REFICULOENDOTHELIAL SOC. 1983, 34(4) 279-87
- CHEMICAL ABSTRACTS, VOL. 99, NO. 24, ISSUED 1983 (COLOMBUS, OHIO, USA), J.V. DESIDERO ET AL.: "INTRAPHAGOCYTIC KILLING OF SALMONELLA TYPHIMURIUM BY LIPOSOME-ENCAPSULATED CEPHALOTHIN", SEE ABSTRACT NO. 200449I, J. INFECT. DIS., 1983, 148(3), PP. 562-70
- CHEMICAL ABSTRACTS, VOL. 102, NO. 24, ISSUED 1984 (COLOMBUS, OHIO, USA) N. POURKAVOOS ET AL.: "THE PROTECTION OF B-LACTAM ANTIBIOTICS FROM ENZYMATIC INACTIVATION BY ENTRAPMENT WITHIN LIPOSOMES", SEE ABSTRACT NO. 209213R, BIOPHARM. PHARMACO. KINETICS, EUR. CONGR., 2ND VOLUME 1, PP. 547-54

## Description

This application is a continuation-in-part of copending U.S. Patent Application Ser. No. 196,913 filed May 20, 1988.

### Field of the Invention

This invention is concerned with high ratio active agent:lipid complexes, their preparation and use. Particular reference is made to cephalosporin:lipid complex and iodinated contrast agent:lipid complex. More particularly disclosed is a high ratio cephalosporin:lipid complex comprising a rigid lipid and a cephalosporin. A method of preparation of high active agent:lipid complexes is also disclosed. Use of cephalosporin:lipid complex in bacterial prophylaxis, and particularly bacteremia prophylaxis, including a method of preventing bacterial infection in an animal over an extended period comprising the step of administering to said animal a high ratio cephalosporin:lipid complex is further disclosed. In one embodiment this invention includes such prophylaxis by maintaining over an extended period an available drug level of at least about a minimum inhibitory concentration. Another embodiment is the use of high ratio iodinated contrast agent:lipid complex in X-ray applications.

### Background of the Invention

A basic element of the practice of therapeutics is the administration of active agents to subjects of treatment. In many situations the problem of administration is one of delivering enough of an active agent in an efficient manner to the sight of action. Liposome encapsulation has, in some instances, offered effective delivery of active agents. However particular active agents have not been efficiently delivered or not delivered in high enough concentrations or in concentrations relative to accompanying lipid that entailed excessive lipid administration. Thus a method of forming a high ratio active agent:lipid complex is of great interest.

Active agents (also termed bioactive agents) are for example, a drugs, hormones, proteins, dyes, vitamins, or imaging agents. As used in the present invention, the term active agent is understood to include any compound having biological activity; e.g., drugs and other therapeutic agents such as peptides, hormones, toxins, enzymes, neurotransmitters, lipoproteins, glycoproteins, immunomodulators, immunoglobulins, polysaccharides, cell receptor binding molecules, nucleic acids, polynucleotides, and the like, as well as including biological tracer substances such as dyes, radio-opaque or radio-contrast agents for X-ray imaging (collectively "contrast agents"), and fluorescent agents.

Prophylactic use of antimicrobial agents is a widely discussed course of pharmaceutical treatment, e.g., "Prophylactic Use of Antimicrobial Agents in Adult Patients", Mayo Clin Proc, 62: 1137-1141 (1987). In appropriate cases, rheumatic fever, bubonic plague, malaria and other diseases are treated prophylactically. A particularly well recognized area of antimicrobial, and especially antibacterial, prophylaxis is the preoperative use of antimicrobial agents. Generally, it has been a concomitant of surgical prophylaxis that the antimicrobial agent be given only during or at most no more than about three hours before surgery.

A central consideration in the administration of prophylactic drug therapy is the ability and ease with which a "minimum inhibitory concentration" ("MIC") of a particular antimicrobial agent may be established and the interval over which such level may be maintained in a subject animal. An MIC is defined as the lowest level of a particular antimicrobial agent (whether antibacterial, anti-infective or other) that will prevent proliferation of the pathogenic organism being treated. An MIC may be established in a physiological fluid such as cerebro-spinal fluid, serum or plasma or in tissue, and particularly tissue at the site of infection (such as the reticulo-endothelial system in disseminated infections).

Conventionally, the MIC for a particular organism is measured in an in vitro system, and is expressed in terms of the amount of drug (by weight) per amount (usually volume) of material in which the drug is dispersed. For a drug to exert a therapeutic or prophylactic effect in vivo the concentration of such drug in vivo will usually be required to be about equal to or greater than the MIC as measured in vitro. Furthermore, the greater the concentration of drug above the MIC and the greater the period of time the in vivo drug level exceeds the MIC, the greater the therapeutic response.

To express the parameter of time over which an in vivo plasma concentration of antibiotic equals or exceeds the MIC of a challenging pathogen the term T greater than MICₚ will be used and written as Tₚ.

To express the parameter of time over which an in vivo tissue concentration of antibiotic equals or exceeds the MIC of a challenging pathogen the term T greater than MICₜ will be used and written as Tₜ.

Liposomes are completely closed lipid bilayer membranes containing an entrapped aqueous volume. Liposomes may be unilamellar vesicles (possessing a single bilayer membrane ) or multilameller vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 13:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles.

Unilamellar vesicles may be produced using an extrusion apparatus by a method described in Cullis et al., PCT Application No. WO 86/00238, published January 16, 1986, entitled "Extrusion Technique for Producing Unilamellar Vesicles" incorporated herein by reference. Vesicles made by this technique, called LUVETS, are extruded under pressure through a membrane filter. LUVETs, being usually of about 500nm diameter or less, end frequently about 100nm, are preferred liposomes of the instant invention. LUVETs will be understood to be included in the term "unilamellar vesicle".

Another class of liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4558,578 to Fountain, et al. and frozen and thawed multilamellar vesicles (FATMLV) wherein the vesicles are exposed to at least one freeze and thaw cycle; this procedure is described in Bally et al., PCT Publication No. 87/00043, January 15, 1987, entitled "Multilamellar Liposomes Having Improved Trapping Efficiencies". The teachings of these references as to preparation and use of liposomes are incorporated herein by reference.

Liposomes with a diameter of about 500nm or less are termed "small liposomes". Similarly, the cephalosporin:lipid complexes of this invention will be termed "small" at a diameter of about 500nm or less. Diameter in describing a population of liposomes or vesicles will be understood to reflect a range of diameters.

The cephalosporin:lipid complexes of this invention are in the form of liposomes at lower drug to lipid ratios. At higher drug to lipid ratios the complexes appear to retain the bilayer organization of liposomes but, in electronmicrograph, may be other than completely closed. The term "complex" is to be understood to encompass both liposomes as well as the higher ratio cephalosporin:lipid entities even if such entities differ from liposomes in having incompletely closed bilayers or other anomalies.

Iodinated contrast agents also form complexes with lipids as an aspect of this invention and exhibit high capture volumes of up to about 20ul/umole lipid. These appear to be in the form of liposomes and are predominately unilamellar and uniform in size at about 0.5 microns. Prior to this invention, contrast agent:lipid ratios of about 2.7:1 were reported as to non-ionic iodinated contrast agents and 1:1 as to negatively charged iodinated contrast agents (e.g., diatrizoate).

Over the past ten years, there have been several groups attempting to develop particulate contrast agents which would specifically opacify organs of the RE system, Seltzer, S.E., Liposomes as Drug Carriers, Gregoriadis, G., ed., pp509-525 (John Wiley and Sons Ltd. New York 1988). One such application involves the detection of metastatic lesions in the liver by x-ray computed tomography (CT). Conventionally a patient is dosed with a water-soluble contrast agent before CT scanning, but because the agent has essentially equal access to both normal and tumor tissue, there is minimal if any contrast between the two tissues. However, if a patient were dosed with particulate contrast agent, the Kupffer cells of the liver would phagocytose the agent causing the cells to become opaque leaving the tumor cells dark. Several particulate agents have been developed and used in humans but these agents have some associated toxicities presumably due to their extended tissue residence time. Particulate contrast agents are reported in Cohen, Z., et al J. Comput. Assist. Tomogr., 5:843-7 (1981); Miller, D.L., et al., AJR, 143:235-243 (1984); Longino, M.A., et al., J. Comput. Assist. Tomogr., 7:775-9 (1983); Mattrey, R.F., et al., Radiology, 145:755-8, (1983); Violante, M.R., et al., Invest. Radiol., 16:40-5 (1981). Another means of achieving a particulate contrast agent is entrapment of the agent in liposomes, Havron, H.A., et al., Radiology, 140:507-11 (1981). The lipid dose required by these preparations is too high for application to humans. The highest reported iodine:lipid ratio in the literature for diatrizoate, the most commonly used contrast agent, is about 1:1 (wt/wt).

In some applications admixing a lipid with cholesterol will reduce the entrapment of cephalosporin. This is potentially the result of cholesterol dispersing in the lipid bilayer and decreasing bilayer rigidity. Lipids are characterized in having a hydrophobic "tail" region and a hydrophilic "head" region. The structure of the membrane bilayer is such that the hydrophobic (nonpolar) "tails" of the lipid monolayers orient toward the center of the bilayer while the hydrophilic "heads" orient toward the aqueous phase. The tail region may be comprised of fatty acyl chains (also denoted as carbon chains), or a single such chain as in the case of alpha-tocopherol hemi-succinate. Examples of lipids are the phospholipids such as phosphatidylcholine (PC) (egg phosphatidylcholine is denoted EPC), phosphatidylethanolamine (PE), phosphatidylserine (PS), phosphatidylglycerol (PG), phosphatidic acid (PA), phosphatidylinositol (PI), sphingomyelin (SPM), and the like, alone or in combination and particularly in hydrogenated or saturated form of the carbon chain. The phospholipids can be synthetic or derived from natural sources such as egg or soy. Synthetic phospholipids include dymyristoylphosphatidylcholine (DMPC) and dimyristoylphosphatidylglycerol (DMPG). The preferred lipids of this invention are those with saturated carbon chains of at least about 16 carbon units as well as nonphospholipids such as digalactosyldiglyceride. They may also contain organic acid derivatives of sterols such as cholesterol hemisuccinate (CHS), and the like. Organic acid derivatives of tocopherols may also be used as liposome-forming ingredients, such as alpha-tocopherol hemisuccinate (THS). Both CHS- end THS-containing liposomes and their tris salt forms may generally be prepared by any method known in the art for preparing liposomes containing these sterols. In particular, see the procedures of Janoff, et al., U.S. Pat. No. 4,721,612 issued January 26, 1988, entitled "Steroidal Liposomes," and Janoff, et al., PCT Publication No. WO 87/02219, published April 23, 1987, entitled "Alpha-Tocopherol Based Vesicles," filed September 24, 1986, respectively. Additional known lipids are glycolipids.

### Sumary of the Invention

In a broad aspect this invention presents a method of preparing high ratio active agent:lipid complex comprising a lipid and an active agent comprising the step of evaporating to monophase but not to substantial dryness a mixture of lipid and active agent in a monophasic solvent system comprising aqueous phase, aqueous miscible organic solvent and lipid.

More particularly the present Invention provides the method claimed in claims 1 to 3.

This invention includes a high ratio cephalosporin:lipid complex comprising a lipid and a cephalosporin, and in one embodiment the lipid is a rigid lipid such as wherein the lipid consists of a head group and two carbon chains said chains are saturated and of a length of at least about 16 carbon units. One rigid lipid is dipalmitoylphosphatidylcholine.

In a particular embodiments of the high ratio cephalosporin:lipid complex the cephalosporin comprises at least 20%, 30%, 40% or 50% of the complex (w/w -- designating the weight of a component as to the total weight). In one embodiment the lipid is dipalmitoylphosphatidylcholine, and potentially up to about 80% (w/w) of the complex. In yet another embodiment of the cephalosporin:lipid complex the lipid comprises of a head group and two carbon chains said chains are saturated and of a length of at least about 16 carbon units.

In particular embodiments the cephalosporin of the high ratio cephalosporin:lipid complex is cefazolin, cephapirin, cephalothin, cefaclor, cephaloridine, cephoxazole, cefoxitin, cephradine, cephalexin, cephaloglycine, cefuroxime, cefmenoxime, or cephalonium, particularly cefazolin.

The invention also includes a method of preparing high ratio cephalosporin:lipid complex comprising a rigid lipid and a cephalosporin comprising the step of evaporating to monophase but not to substantial dryness a mixture of rigid lipid and cephalosporin in a monophasic solvent system comprising aqueous phase, aqueous miscible organic solvent and lipid. In one embodiment to the method the monophasic solvent system is about equal amounts (v/v) of (a) aqueous phase of drug, (b) ethanol and (c) chloroform and said lipid.

In a particular embodiment the method comprises the step of evaporating to monophase but not to substantial dryness a mixture of said lipid and cephalosporin in a solvent system comprising about equal amounts (v/v) aqueous phase
(drug-and-lipid):ethanol:chloroform, including evaporating solvent by positive pressure at about 30 to about 37°C. In the practice of one embodiment of this invention the method comprises about two administrations each day or less. In further embodiments the method comprises maintaining over an extended period an available cephalosporin level of at least a minimum inhibitory concentration ("MIC") of the cephalosporin in an animal, and specifically wherein the the cephalosporin level is at least about 4 times the MIC. In some embodiments the MIC is measured as a blood plasma or serum level or tissue level. In some embodiments administration is intramuscularly, intraperitoneally, intravenously or subcutaneously. In the practice of this method the cephalosporin may comprise any or all of cefazolin, cephapirin, cephalothin, cefaclor, cephaloridine, cephoxazole, cefoxitin, cephradine, cephalexin, cephaloglycine, cefuroxime, cefmenoxime or cephalonium. In certain embodiments of the method wherein the cephalosporin comprises cefazolin the method further comprises administering said cefazolin at a dosage of about 20-150mg base weight per kilogram animal body weight per day or the maintained available cephalosporin level of cefazolin is at least about 0.5, 5, or 50ug (base weight)/ml plasma or at least about 0.5ug (base weight)/mg tissue (RES tissue such as liver or spleen).

In particular embodiments of the method the resulting cephalosporin:lipid complex is at least about 30%, 40%, or 50% (w/w) cephalosporin. In some embodiments of the method wherein the lipid is comprised of a head group and two carbon chains said chains being saturated and of a length of at least about 16 carbon units or the lipid is dipalmitoylphosphatidylcholine, such as up to about 80% (w/w) of the complex. In the practice of the method the cephalosporin is in particular embodiments cefazolin, cephapirin, cephalothin, cefaclor, cephaloridine, cephoxazole, cefoxitin, cephradine, cephalexin, cephaloglycine, cefuroxime, cefmenoxime or cephalonium and particularly cefazolin.

The method of this invention yet further includes a method of bacterial infection prophylaxis in an animal, including a human, comprising the step of administering to said animal a bacterial infection prophylaxis effective amount of cephalosporin:lipid complex, and in one embodiment wherein the complex is a high ratio cephalosporin:lipid complex, the lipid is a rigid lipid.

A particular embodiment of the prophylaxis method of this invention is to bacteremia. In the practice of one embodiment of this invention the method comprises about two administrations each day or less. In further embodiments the method comprises maintaining over an extended period an available cephalosporin level of at least a minimum inhibitory concentration ("MIC") of the cephalosporin in an animal, and specifically wherein the the cephalosporin level is at least about 4 times the MIC. In some embodiments the MIC is measured as a blood plasma or serum level or tissue level. In some embodiments administration is intramuscularly, intraperitoneally, intravenously or subcutaneously. In the practice of this method the cephalosporin may comprise any or all of cefazolin, cephapirin, cephalothin, cefaclor, cephaloridine, cephoxazole, cefoxitin, cephradine, cephalexin, cephaloglycine, cefuroxime, cefmenoxime or cephalonium. In certain embodiments of the method wherein the cephalosporin comprises cefazolin the method further comprises administering said cefazolin at a dosage of about 20-150mg base weight per kilogram animal body weight per day or the maintained available cephalosporin level of cefazolin is at least about 0.5, 5, or 50ug (base weight)/ml plasma or at least about 0.5ug (base weight)/mg tissue (RES tissue such as liver or spleen).

In particular embodiments of the method the resulting cephalosporin:lipid complex is at least about 30%, 40%, or 50% (w/w) cephalosporin. In some embodiments of the method wherein the lipid is comprised of a head group and two carbon chains said chains being saturated and of a length of at least about 16 carbon units or the lipid is dipalmitoylphosphatidylcholine, such as up to about 80% (w/w) of the complex. In the practice of the method the cephalosporin is in particular embodiments cefazolin, cephapirin, cephalothin, cefaclor, cephaloridine, cephoxazole, cefoxitin, cephradine, cephalexin, cephaloglycine, cefuroxime cephmenoxime or cephalonium and particularly cefazolin.

A particular embodiment of the prophylaxis method of this invention is to disseminated bacterial infections involving the reticulo-endothelial system. In the practice of one embodiment of this invention the method comprises about two administrations each day or less. In further embodiments the method comprises maintaining over an extended period an available cephalosporin level of at least a minimum inhibitory concentration ("MIC") of the cephalosporin in an animal, and specifically wherein the the cephalosporin level is at least about 4 times the MIC. In some embodiments the MIC is measured as a blood plasma or serum level or tissue level (such as RES tissue, e.g., liver or spleen). In some embodiments administration is intramuscularly, intraperitoneally, intravenously or subcutaneously. In the practice of this method the cephalosporin may comprise any or all of cefazolin, cephapirin, cephalothin, cefaclor, cephaloridine, cephoxazole, cefoxitin, cephradine, cephalexin, cephaloglycine, cefuroxime, cefmenoxime or cephalonium. In certain embodiments of the method wherein the cephalosporin comprises cefazolin the method further comprises administering said cefazolin at a dosage of about 20-150mg base weight per kilogram animal body weight per day or the maintained available cephalosporin level of cefazolin is at least about 0.5, 5, or 50ug (base weight)/ml plasma or at least about 0.5ug (base weight)/mg tissue (RES tissue such as liver or spleen).

In particular embodiments of the method the resulting cephalosporin:lipid complex is at least about 30%, 40%, or 50% (w/w) cephalosporin. In some embodiments of the method wherein the lipid is comprised of a head group and two carbon chains said chains being saturated and of a length of at least about 16 carbon units or the lipid is dipalmitoylphosphatidylcholine, such as up to about 80% (w/w) of the complex. In the practice of the method the cephalosporin is in particular embodiments cefazolin, cephapirin, cephalothin, cefaclor, cephaloridine, cephoxazole, cefoxitin, cephradine, cephalexin, cephaloglycine, cefuroxime, cefmenoxime or cephalonium and particularly cefazolin. A particular embodiment comprises prophylaxis for the bacteria Mycobacterium sp. or Salmonella sp. A further embodiment of this invention is prophylaxis directed to bacterial osteomyelitis.

An important aspect of this invention is a high ratio iodinated contrast agent:lipid complex comprising a lipid and an iodinated contrast agent, particularly wherein the lipid is a rigid lipid but also with unsaturated lipid such as phosphatidylcholine. In some embodiments the contrast agent is at least about 2:1 (Iodine w/w), or at least about 4:1 (Iodine w/w) or at least about 8:1 (Iodine w/w). In some instances the lipid comprises a head group and two carbon chains said chains are saturated and of a length of at least about 16 carbon units such as dipalmitoylphosphatidylcholine which comprises as little as about 20 to about 7% (w/w) of the complex. Useful contrast agents are without limitation diatrizoate, iocetamic acid, iodamide, iodoximate, iohexol, iopamide, iopamidol, iopanoic acid, iophendylate, iothalamate, iothalamic acid, ipodate, metrizamide or tyropanate and salts thereof.

This invention also includes a method of preparing high ratio active agent:lipid complex wherein the active agent is an iodinated contrast agent comprising the step of evaporating to monophase but not to substantial dryness a mixture of lipid and contrast agent in a monophasic solvent system comprising aqueous phase, aqueous miscible organic solvent and lipid. In one embodiment of this method wherein the monophasic solvent system is about equal amounts (v/v) of (a) aqueous phase of contrast agent (b) ethanol and (c) chloroform and said lipid. Also, optionally, further comprises evaporating solvent by positive pressure at about 30 to about 75°C. By this method the contrast agent of the resulting contrast agent:lipid complex is at least about 2:1 (Iodine w/w) or at least about 4:1(Iodine w/w) or at least about 8:1 (Iodine w/w/). Rigid lipids are used in this method such as wherein the lipid comprises of a head group and two carbon chains said chains being saturated and of a length of at least about 16 carbon units, particularly wherein said dipalmitoylphosphatidylcholine comprises as little as about 20% to about 7% (w/w) of the complex. Also useful in the method is wherein the lipid comprises phosphatidylcholine such as egg or soy phosphatidylcholine. Particular contrast agents of the method are diatrizoate, iocetamic acid, iodamide, iodoximate, iopamide, iopamidol, iopanoic acid, iophendylate, iothalamate, iothalamic acid, ipodate calcium, ipodate sodium, metrizamide or tyropanate and salts thereof.

An additional aspect of the invention is a method of increasing X-ray density in an animal, including a human, comprising the step of administering to said animal an X-ray density increasing effective amount of iodinated contrast agent:lipid complex such as wherein the lipid is a rigid lipid as well as wherein the contrast agent is diatrizoate, iocetamic acid, iodamide, iodoximate, iopamide, iopamidol, iopanoic acid, iophendylate, iothalamate, iothalamic acid, ipodate calcium, ipodate sodium, metrizamide or tyropanate and salts thereof. In some applications the contrast agent comprises diatrizoate further comprising administering said diatrizoate at a dosage of about 500mg or about 1 gm iodine per kilogram animal body weight per day. One embodiment is wherein the increase in X-ray density is to the liver.

### Brief Description of the Drawings

Figure 1 shows the declining entrapment of cephapirin sodium in egg phosphatidylcholine complex vs. drug concentration.

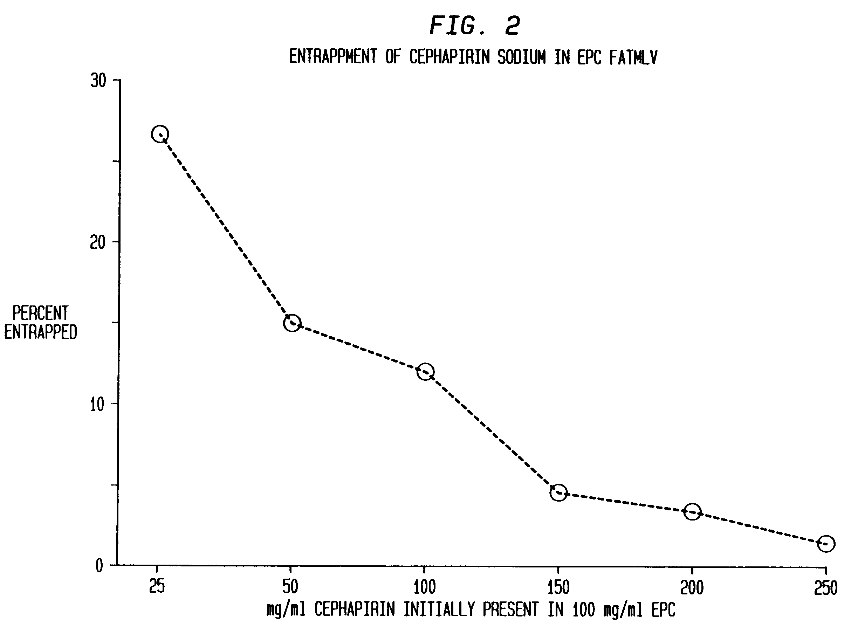

Figure 2 shows the entrapment of cephapirin sodium in egg phosphatidylcholine complex vs. drug concentration.

Figure 3 shows flexibility profiles of EPC, CHS and alpha-THS in defining rigid lipids.

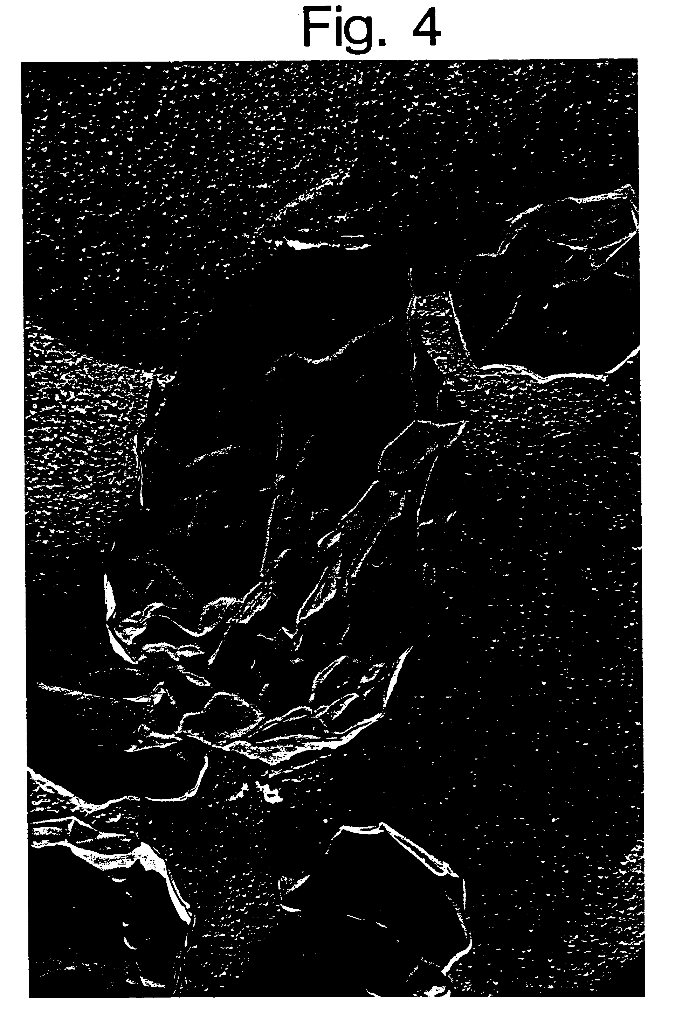

Figure 4 shows high ratio 1:2 (w/w) cefazolin:dipalmitoylphosphatidylcholine complex in electronmicrograph.

Figure 5 shows freeze-fracture electron micrographs of cephapirin-egg phosphatidylcholine complexes made by either a standard or modified MPV process in which (a) the drug-lipid film was dried to apparent dryness (standard process) or (b) the drug-lipid film followed increased initial aqueous component and maintained residual water -- which may contain some residual organic solvent (modified MPV process).

Figure 6 shows the effect of initial aqueous volume in cephalosporin:lipid mixture on association of cephapirin with dipalmitoylphosphatidylcholine.

Figure 7 shows the effect of increasing lipid:cephapirin molar ratio on association of cephapirin with dipalmitoylphosphatidylcholine.

Figure 8 shows serum levels of cephapirin activity in mice at intervals after injection of free cephapirin in the form of cephalosporin:lipid complex cephapirin.

Figure 9 shows serum levels of cefazolin activity in mice at intervals after injection of free cefazolin in the form of cephalosporin:lipid complex cefazolin.

Figure 10 shows the effect of cephapirin either free or in cephalosporin:lipid complex on abcess size.

Figure 11 shows the effect of cephapirin either free or in cephalosporin:lipid complex on mortality of mice after challenge by Salmonella typhimurium.

Figure 12 discloses the retention of contrast agent in the liver in high ratio complex form versus free contrast agent.

### Detailed Description of the Invention

Experiments in animals have shown that cephalosporin associated with lipid, such as in a liposome or in a cephalosporin:lipid complex, can dramatically alter the distribution of cephalosporin in the body and their rate of clearance. These pharmacokinetic differences, as well as other, less well understood effects, can result in prolonged prophylactic levels of cephalosporin, reduced toxicity and/or enhanced efficacy of the cephalosporin associated with a lipid.

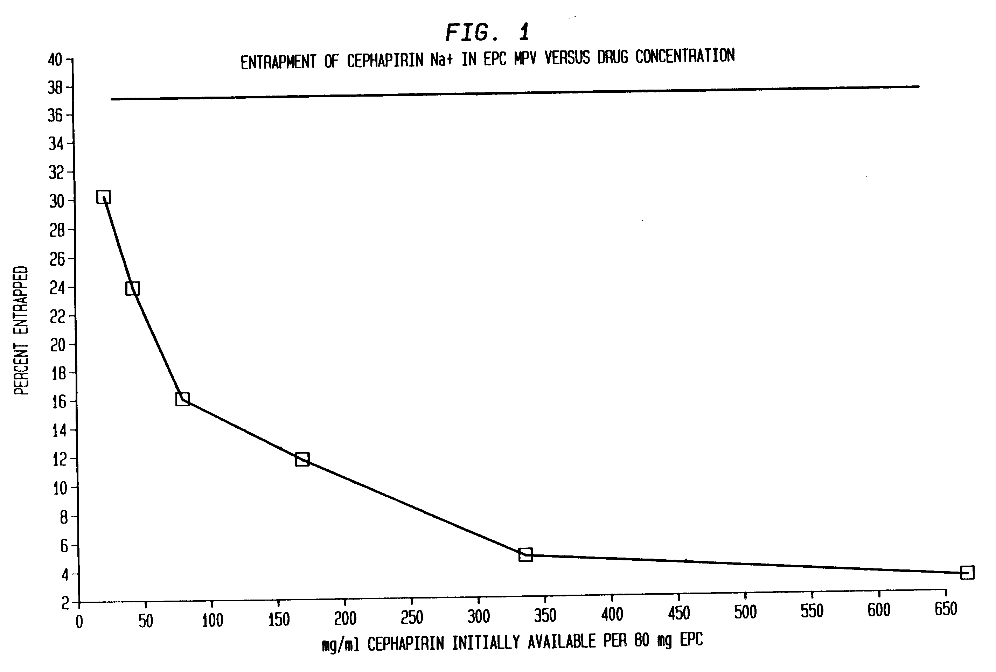

However, the entrapment of cephalosporins into liposomes (or complexes) is typically a low percentage of available drug. As shown in Fig. 1 the entrapment of the cephalosporin cephapirin in the lipid egg phosphatidylcholine decreases with increasing amounts of cephalosporin. The decreasing entrapment indicates a deleterious effect on entrapment by high concentrations of cephalosporin.

"High ratio cephalosporin:lipid complex" is at least about 20% cephalosporin as compared to lipid (w/w). In some embodiments with cephalosporin these are of at least about 30%, and up to about 50% or more of cephalosporin (w/w). High ratio cephalosporin:lipid is particularly useful in cephalosporin therapy for several reasons. First with conventional ratio cephalosporin:lipid the physical mass of the unit lipid dosage is inconvenient for administration if not prohibitive of administration. A typical cephalosporin dose is 4g/day. For an adult at previously reported ratios of 0.14g (cephalosporin):1g (lipid) the unit dosage would be about 32g of drug and lipid. The usual mode of administration would require admixture with a pharmaceutically acceptable carrier resulting in a unit dosage that was at best unwieldly. Reducing this bolus by a substantial amount of up to about 75% or more is a particular advantage of this invention. Second, toxicity may be associated with administration of large amounts of lipid which is reduced by reducing the amount of lipid administered. Third, the cost of a concentrated dose is substantially lessened by reducing the required amount of lipid. An additional benefit of the instant invention is the high percentage of drug available in a drug:lipid complex preparatory mixture is entrapped in the final complex, which further reduces the coat of the final cephalosporin:lipid preparation.

High ratio contrast agent:lipid complex will be in excess of about 2.7:1 )(Iodine w/w) for nonionic contrast agents and in excess of about 1:1 (Iodine:lipid wt/wt) for ionic contrast agents (negatively charged).

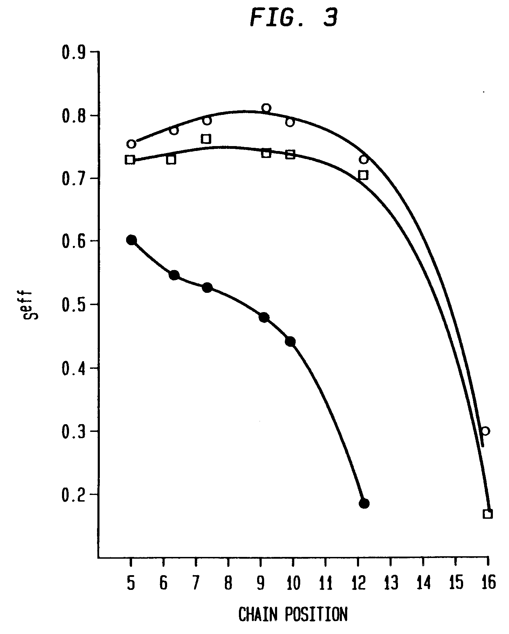

In the present invention, the term rigid lipid as used herein shall mean lipids of a flexibility about equal to or less than that of egg phosphatidylcholine Rigidity is easily determined by methods well known in the art. For example, Fig. 3 expresses rigidity in the spin labeling of lipids in complexes via a doxyl reporter group. As represented in Fig. 3 rigid lipids are those with an order parameter about equal to that of EPC or greater therefore closer to an order parameter of 1 (above of EPC in Fig 3). The order parameters were obtained by spin labeling the vesicles with a series of doxyl stearic acids where the doxyl reporter group was present at different positions along the fatty acid chain. Spectra obtained showed no evidence of unincorporated label.

Particularly included as rigid lipids are those with a head group and two carbon chains said chains being of a length of at least about 16 carbon units where the chains are saturated. Such lipids as dipalmitylphosphatidylcholine (DPPC) and distearoylphosphatidylcholine are in this group. Also, cholesterol hemisuccinate tris(hydroxymethyl)aminomethane (CHSₜᵣᵢₛ) and the like are rigid lipids.

The preparation of high ratio active agent:lipid complex, such as with either a cephalosporin or a contrast agent may be accomplished by a modification of the method described in U.S. Patent No. 4,588,578 entitled "Lipid Vesicles Prepared in a Monophase" the teachings of which are incorporated herein by reference. The vesicles prepared in a monophase are referred to as monophasic vesicles or "MPVs". Monophasic vesicles arise from a novel preparatory process using a monophasic solvent system. The monophasic solvent system is comprised of three components -- (1) lipid, (2) aqueous miscible organic solvent and (3) aqueous component. To produce the novel monophasic vesicle these components are mixed to form a monophase prior to removal of the organic solvent. After removal of the organic solvent a drug-lipid film is formed which upon hydration forms monophasic vesicles.

The modified MPV process entails practicing the MPV process at a relatively high initial aqueous volume generally from about 8:1 to about 1:1 (v/v) of aqueous active agent (e.g.,cephalosporin) to organic solvent. In the practice of the method of forming high ratio active agent:lipid complex the initial mixture of the monophasic solvent system must pass through a monophase stage whether or not the solvent system begins as a monophase. The term "monophase" shall mean a combination of 2 or more solvents which upon mixing result in a clear solution of constant composition. In practice 2 immiscible solvents which upon mixing form a biphasic system are converted into monophase by the addition of a third solvent that is miscible in both of the original solvents. For example chloroform and water form a biphasic system upon mixing, but form a monophase upon addition of sufficient ethanol.

The central modification of the MPV process is the step of maintaining the active agent (such as cephalosporin or contrast agent) and lipid as hydrated at all times. This hydrated material is the complex of the invention which is usefully diluted into concentrations more convenient for pharmaceutical unit dosage form. The modification of maintaining the material as hydrated then avoids the usual end point of removing all of the aqueous component and forming an active agent:lipid film. That the monophase is hydrated is easily determined simply by observing the material during the evaporative step. The presence of a gel like material indicates that water is still present, whereas if all water is removed a lipid-active agent film will be formed on the vessel containing the material. The foregoing procedure markedly increased the percentage of active agent entrapped relative to active agent available. This procedure also resulted in increasing in final active agent:lipid ratio resulting in the high ratio active agent:lipid complex of the instant invention. Utilizing the foregoing method and rigid lipids, entrapment or association of up to about 60 or 70% of the initial active agent such as cephalosporin may be obtained compared to the entrapment or association of about one-half that amount without the instant method. Furthermore the final active agent:lipid ratio in the case of cephapirin has increased from about 1:7 (w/w) (cephapirin, egg phosphatidylcholine, and MPV method) to about to better than about 1:1 including about 1.2:1.

Final iodine:lipid ratios for contrast agents are reported for iopamidol C. Musu, et al., Invest Radiol, 23(Supp.1): S126-129 (1988).

By the method of this invention ratios at least of about 2:1 (Iodine weight/weight) or about 4:1 (Iodine w/w), 6:1 (Iodine w/w) and up to even about 8:1 (Iodine w/w) are obtained. 8:1 diatrizoate:lipid complex was prepared. With iodine contrast agents the the use of rigid lipids is advantageous as is the use of unsaturated phosphatidylcholines. It is believed that rigid lipids yield complexes more stable in sera whereas unsaturated phosphatidylcholines yield higher iodine:lipid ratios in complex.

The bench mark in obtaining X-ray contrast with iodinated agents is the amount of iodine delivered. For diatrizoate the iodine to diatrizoate ratio is 1 gram of iodine for 1.623 grams of diatrizoate. In many applications administration of from about 500 to about 1000 mg of iodine per kilogram body weight is required. However depending on various factors such as the concentration of iodine at the site of imaging or the excretion of contrast agent prior to biological availability dosages outside these limits may be utilized. Without being bound by any theory it is thought that since particulates such as liposomes and the complexes of this invention are phagocytosed by liver cells the intracellular space is labeled preferentially to the interstitial space. As will be seen below the contrast agent as disposed by the complexes of this invention facilitate the visualization of liver tumors in particular.

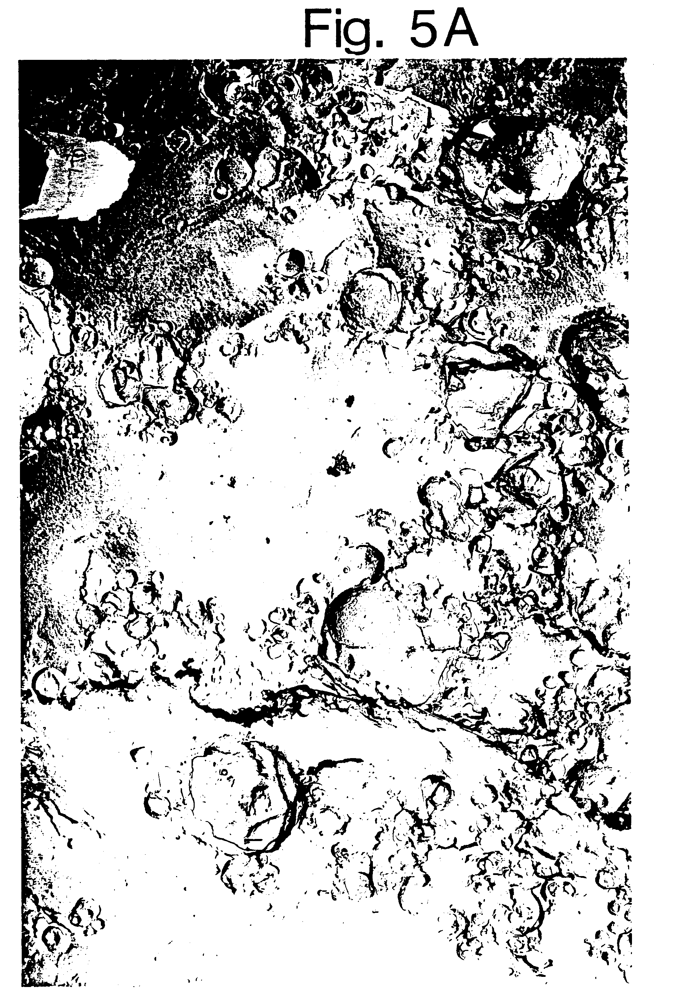

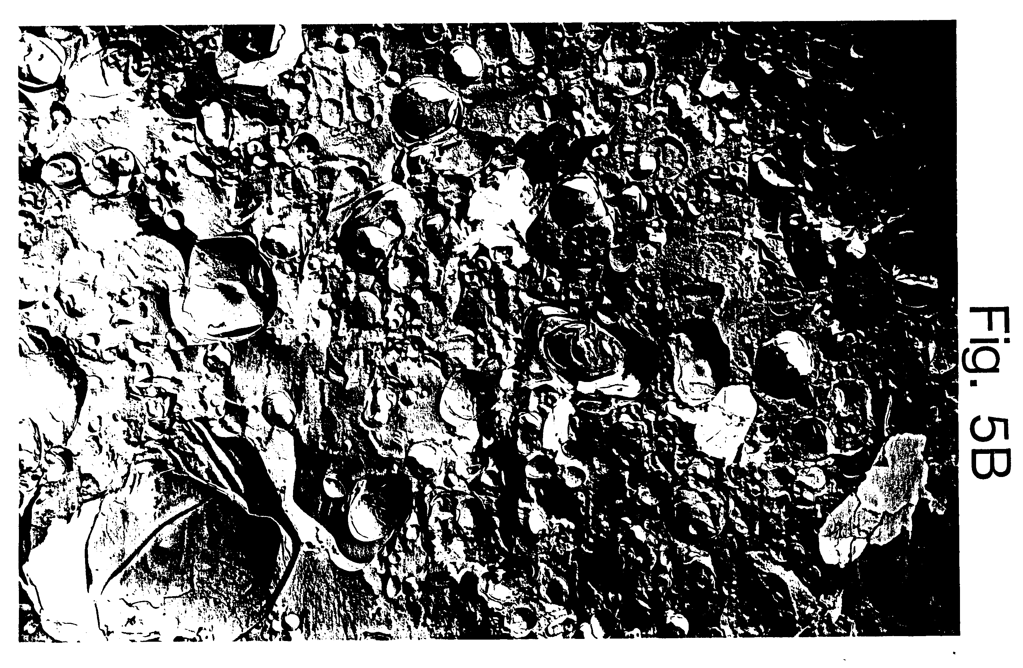

Figure 5A and 5B are a freeze-fracture electron micrographs of cephapirin-egg phosphatidylcholine complexes made by either a standard or modified MPV process in which (a) the cephalosporin-lipid film was dried to apparent dryness (standard process) or (b) the cephalosporin-lipid film followed increased initial aqueous component and maintained residual water (modified MPV process). The complexes in (a) appear irregular and distinct from known cephalosporin:lipid complexes, while in (b) the complexes appear to be typical EPC MPVs. Without being bound by any particular theory it appears that as the conventional MPV process entails removing all liquid from the aqueous drug-lipid-organic solvent such removal should be avoided Water removal to dryness results in low drug entrapment and low ratio cephalosporin:lipid complex as in Fig. 5A. By maintaining a residual aqueous phase the entrapment or association roughly doubled, forming the complexes of Fig. 5B.

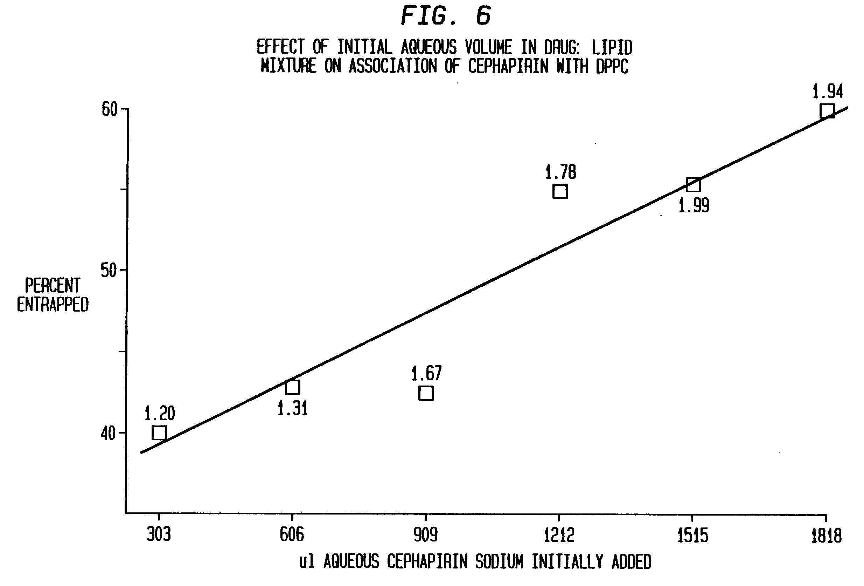

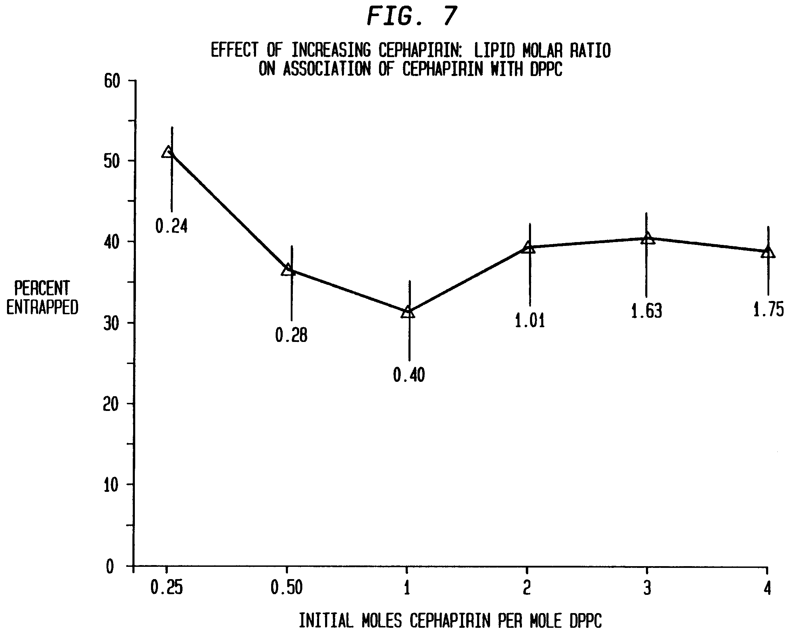

Figure 6 shows the effect of increasing initial aqueous volume on the entrapment or association of cephapirin in DPPC complex while holding the cephalosporin:lipid constant at 1:3. Fig. 6 discloses that increasing the initial aqueous volume by 6-fold increases from about 1:1 to 2:1 the cephalosporin:lipid ratio. In order to eliminate the possibility that this increase in cephalosporin:lipid ratio was not solely due to increase in aqueous volume rather than a decrease in drug concentration entrapment using the highest aqueous volume tested in Fig. 1 was tested while holding constant this volume as well as lipid amount. Fig. 7 represents the results of a test of entrapment while holding volume and lipid constant. Fig. 7 discloses that no dramatic change in entrapment as the initial cephalosporin:lipid ratio is increased, i.e., more drug added.

The preferred process of the instant invention differs from the MPV process in several ways. First, the initial combination of drug and lipid in a solvent system may not consist of a monophase. However the solvent system passes through a monophase at some point during solvent removal in order to achieve high entrapment or association and result in high active agent:lipid ratio. Regardless of the system used both active agent (also termed drug) and lipid should be completely dissolved in their respective phases. When the active agent is a contrast agent and the lipid a rigid lipid, temperatures about 10°C above the transition temperature of the lipid are useful. Next, an evaporative procedure such as positive pressure should be used rather than vacuum rotary evaporation in order to limit the loss of water during the solvent removal process. Using a paddle stirrer during this step is advantageous. In the preferred process the rate of solvent removal is monitored so as to avoid solvent removal which is too rapid to permit formation of a distinct monophase. This rate is closely associated with temperature in that higher temperature results in faster solvent removal. It is also desirable to remove greatest amount of organic solvent while retaining the greatest amount of water.

While a number of organic solvents are acceptable in the MPV process, in the instant process combinations of solvents must cosolubilize both drug and lipid and form a monophase during the process. Ethanol is substantially the preferred aqueous miscible organic solvent for high entrapment or association and high ratio active agent:lipid complex. An advantage of ethanol as the aqueous miscible organic solvent is that in vivo high residuals of ethanol do not appear to have deleterious effect on the complex as judged by mouse I.V. pharmacokinetic experiments.

One aspect of this invention is bacterial infection prophylaxis in an animal by maintaining, over an extended period, an available drug level of at least about an MIC level of a cephalosporin in an animal comprising the step of administering to said animal a lipid:cephalosporin complex and preferably a high ratio complex in an increased administration-interval regimen.

The "extended period" for maintenance of prophylactic levels (Tₜ or Tₚ of a particular cephalosporin (or other antibacterial or anti-infective) shall be understood to mean a period in excess of at least about twice that time period over which a drug level is about equal to or exceeding an MICₚ or MICₜ that may be maintained by a single administration of an equivalent weight of a free -- that is non-complex -- form of such cephalosporin.

"Available" as referring to the the prophylactically effective level of a cephalosporin (or other antibacterial or anti-infective) shall be understood to mean bioavailable; that is the cephalosporin, whether in a physiological liquid such as cerebro-spinal fluid, serum, or plasma or in tissue, exhibiting antibacterial activity against pathogens as such pathogens which are presented in vivo.

"Drug level of at least a minimum inhibitory concentration" shall be understood to mean a bacterial infection prophylactic level of cephalosporin (or other antibacterial or anti-infective) sufficient to prevent the proliferation an organism in a subject animal. This bacterial infection prophylactic level will be at least about the MIC level. This level is easily determined by a number of in vitro tests of MIC well known to those skilled in the art. The administered dose to establish in vivo such a prophylactically effective level will vary with the absolute drug level to be obtained, the particular drug, the site where the level is to be established and other factors known to those skilled in the art. For animals, including humans, in the prophylaxis of bacterial infection, the prescribing medical professional will ultimately determine the appropriate dosage for a given subject, and this can be expected to vary according to the age, weight, and response of the animal, route of administration as well as the nature and severity of the bacterial challenge. The prophylactic level and hence dosage amount thereby required in liposome-encapsulated form will generally be about that employed for the free therapeutic agent. In some cases, however, it may be necessary to administer doses outside these limits to establish the desired in vivo drug levels. In a specific application such as the prophylaxis of bacteremia, that is a bacterial infection of the blood, a prophylactic level of cephalosporin shall be understood to mean that level that prevents proliferation of the subject bacteria in the blood.

Cephalosporin shall be understood to mean any member of the cephalosporin class of β-lactam antibiotics, including, without limitation, cefazolin, cephapirin, cefaclor, cephaloridine, cephoxazole, cefoxitin, cephaloridine, cephradine, cephlexin, cephaloglycine, cefuroxime, cefmenoxime, and cephalothin and analogues and derivatives thereof.

"Iodinated contrast agents" shall be understood to mean iodinated pharmaceutically acceptable materials with discernable X-ray opacity such as without limitation diatrizoate, iocetamic acid, iodamide, iodoximate, iohexol, iopamide, iopamidol, iopanoic acid, iophendylate, iothalamate, iothalamic acid, ipodate calcium, ipodate sodium, metrizamide or tyropanate and which will be understood to include salts thereof such as meglumine (e.g., diatrizoate meglumine, iodamide meglumine, iodoximate meglumine, iopamide meglumine, iothalamate meglumine) as well as other pharmaceutically acceptable salts and additionally analogues and derivatives thereof.

A free drug may be administered to an animal in an amount sufficient to establish an MIC in the animal, generally throughout the animal or at a site within the animal. The MIC for such drug will be present for a certain amount of time specific to each drug and animal and other well known factors . After a time the drug level will fall below the MIC and the drug must be reädministered to reëstablish the MIC. Thus to continuously maintain a drug level of at least about an MIC, or any given drug level, repeated administrations of the drug must be made to the animal.

Cephalosporin:lipid complex and particularly high ratio cephalosporin:lipid complex is useful in continuously maintaining a drug level of at least an MIC without repeated administrations of drug as compared to free drug. "Cephalosporin: lipid complex" refers to a cephalosporin, associated with a lipid, preferably a rigid lipid. Drug may be adsorbed to the outer surface of a complex or within an aqueous or lipid portion of a complex. As cephalosporin is generally hydrophilic most cephalosporin of a cephalosporin:lipid complex will be within the complex, but this is not critical.

While it is not a limitation of the invention, complexes may be washed to remove active agent such as cephalosporin that is disassociatable from the complex. Since some external cephalosporin is easily separated from the complex upon administration to an animal, it may behave more as free cephalosporin than high ratio cephalosporin:lipid complex. To maximize the complexed dose of cephalosporin while avoiding unnecessary toxicity from disassociatable cephalosporin, in the preferred embodiment, complexes are washed to remove at least about 90% of the disassociatable cephalosporin. Methods of washing complexes are well known in the art and include centrifugation as well as chromatography.

In the practice of this invention for prophylaxis the number of such administrations of drug which is complexed is reduced as compared to free drug that must be administered in equivalent dosage amounts to maintain the same drug level or MIC of drug. The reduction in the number of times a drug must be administered to maintain a given tissue or blood level is necessarily reflected in an increased time interval between administrations of such drug. This is particularly advantageous in prophylexis of infections that have a lengthy period of intracellular survival including disseminated infections involving the reticulo-endothelial system ("RES") such as Salmonella sp. or Mycobacterium sp. particularly Mycobacterium avium intracellulare. The "increased administration-interval regimen" of this invention shall be understood to mean an administration regimen having an interval between administrations of drug of at least two times that interval at which free drug must be reädministered (at equivalent weights and the same drug) to maintain a given drug level. In some therapeutic situations only a single administration of cephalosporin:lipid complex will be required to replace two or more administrations of non-complex drug -- which is essentially an infinite increase in the administration-interval and understood to be within this definition. In treatment of disseminated bacterial infections involving the reticulo-endothelial system the persistence of cephalosporin after administration in cephalosporin:lipid complex form permits efficacy where previously cephalosporin therapy had been ineffective.

"Minimum Inhibitory Concentration" ("MIC") is a term well known in the art refering to the least level of a therapeutic agent, usually expressed as w/w or w/v, which will inhibit the proliferation of a pathogen -- here a bacterium. As used herein the MIC as detected in blood plasma or serum is termed "MICₚ" and understood to refer to a concentration of therapeutic agent in the plasma or serum equal to or greater than the minimum inhibitory concentration of pharmaceutical agent in weight per volume of plasma or serum as determined in vitro for the challenging organism. For cefazolin the MICₚ for many susceptible organisms is about 0.5ug (base weight)/ml (plasma or serum). Cephalosporin:lipid complex cefazolin may be administered to reach this level and further may reach levels of 10 or 100ug/ml or more. Base weight will be used to refer to the weight of the active moiety without including the counterion.

As used herein the MIC as detected in tissue is termed "MICₜ" and understood to refer to a concentration of therapeutic agent in the tissue equal to or greater than the minimum inhibitory concentration of pharmaceutical agent in weight per weight as determined in vitro for the challenging organism. For cefazolin the MICₜ for many susceptible organisms is about 0.5ug (base weight)/mg (spleen). Drug:lipid complex cefazolin may be administered to reach this level and further may reach levels of 10 or 100ug/ml or more.

In the treatment of pathological conditions, the establishing of a prophylactic level of pharmaceutical agent in tissue can also be an effective therapeutic option. Often levels of about 4 or about 8 times the MIC are desired for the most reliable prophylaxis. Such levels are obtainable by the method of the instant invention. Parenteral, such as intravenous, administration of lipid complex pharmaceutical agent such as a cephalosporin results in the prolonged presence of pharmaceutical agent in selected tissue exhibiting pronounced antibacterial activity (Table 1). Experiments show that the tissue levels of cephapirin persist longer than serum or plasma levels and are above the MIC's of most sensitive organisms for an extended period of time, Tₜ. For example, levels greater than about 60ug/gm are present in the spleen for at least about 24 hours, and in the liver for about 24 hours.

Beyond the prophylactic treatment for bacterial infection, the persistence of high ratio cephalosporin complex is particularly important in the treatment of facultative bacterial intracellular infections also termed disseminated bacterial infections involving the reticulo-endothelial system. Facultative refers to pathogens which are capable of surviving inside eukaryotic cells, especially the macrophages of the RES, as well as on an acellular medium. Examples of facultative intracellular organisms are Listeria monocytogenes, Brucella sp. Legionella pneumophilia, Mycobacterium sp. and Salmonella sp. An additional group of bacterial pathogens, the obligate intracellular organisms, will be included with the facultative organisms. The obligate intracellular organisms, such as coxiella burnetii, can only survive intracellularly.

It will be understood that some bacteria are cephalosporin resistant at subtoxic levels of cephalosporin or sensitive to cephalosporin only at toxic levels. Similar resistance may occur with other antibacterial or anti-infective agents. Those skilled in the art recognize that this invention will be applicable to organisms sensitive to cephalosporin or other antibacterial or anti-infective agents. This is determinable by in vitro testing. Organisms such as Salmonella sp. not typically treated with cephalosporin but sensitive in vitro are effectively treated by the instant method.

Prophylactic treatment of bacterial infection is accomplished by the administration of an effective amount of high ratio cephalosporin:lipid complex (or other antibacterial or anti-infective agents). A bacterial infection prophylaxis effective amount will be understood to mean a sufficient amount to achieve the prophylactic response over the desired interval. The bacterial infection prophylaxis effective amount of a given cephalosporin (or other antibacterial or anti-infective agents) will vary with the mode of the administration, the particularities of the recipient, the sensitivity of the target bacteria, and other factors well known in the art.

The duration of prophylaxis from a single dose of cephalosporin:lipid complex (or other antibacterial or anti-infective agents) will vary depending on the specific drug, the animal being treated, the challenging organism, and other factors known to those skilled in the art. In general, a prophylactic tissue level of drug, arising from a single administration of drug, will be maintained in the spleen and liver substantially longer and greater than the Tₜ of the free agent in similar tissue from a single administration. In the liver the complex persisted at least about 2 times and up to about 5 times or more longer than the free drug. In the spleen free drug was below detectable levels whereas the complex drug lasted at least about 24 hours.

The duration required for prophylactic treatment will vary with the condition of the subject animal. In immunocompromised animals prophylaxis is potentially required for the period of immunocompromise. Thus in acquired immunodeficiency (AIDS) subjects the period may be for life while in subjects suffering neutropenia secondary to chemotherapy or immunosuppressed for organ transplant receipt the period of prophylaxis may be more circumscribed. The duration of such periods of prophylactic need is well known to those skilled in the art.

The maintenance of at least a minimum inhibitory concentration of antibiotic in certain organs of the RES in the absence of detectable serum levels may be advantageous for the prevention and treatment of bacteremia in patients at risk or in the treatment or suppression of certain chronic infections (e.g., osteomyelitis, mycobacterium avium intracellulare infection, Salmonella sp. infections).

The reticulo-endothelial system or RES is understood to refer to cells of the body having both endothelial and reticular attributes and showing a common phagocytic behavior toward dyestuffs. Such cells tend to incorporate administered cephalosporin:lipid complex. Included cells are those of the spleen and lymph nodes, Kupffer cells of the liver, alveolar macrophages of the lung, the reticulo-endothelium of bone marrow and the clasmatocytes. In the terminology of this invention bone the marrow and hard tissue will be deemed to be within the RES, and bacterial osteomyelitis to be an infection of the RES.

To determine the bioavailability of cefazolin when administered in a cephalosporin:lipid complex, several infection models were studied.

In general, the serum level and tissue distribution studies for antibiotics were performed in mice by injecting a single intravenous dose of free or encapsulated antibiotic and quantitating the amount of antibacterial activity in the serum or plasma and selected tissues of the animals at intervals thereafter.

The antibacterial activity was measured by an agar well diffusion bioassay, though other assays for antibacterial activity or chemical, or immunological assays for the drug substance are known to those skilled in the art. Briefly, the agar well diffusion bioassay used here were performed by cutting wells into an agar such as Trypticaso Soy Agar (Difco., Detroit, MI) that were previously seeded with a test bacteria such as Bacillus subtilis (ATCC #6633). The wells were filled with a small amount of test material here about 50ul of the test sample, and the agar plates were incubated. Bacillus subtilis incubation was for about 16-24 hours at about 35°C. Growth of a bacterial "lawn" around the well was inhibited by antibiotic present in the agar as diffused from the test sample. The diameters of the zones of inhibited growth around the wells were proportional to the concentration of antibiotic in the test sample and were quantitated by comparison with known, standard antibiotic solutions.

The administration (parenteral) to an animal of a cephalosporin:lipid complex antibiotic such as cefazolin to an animal characteristically resulted in a higher peak serum or plasma level as measured by the concentration of pharmaceutical agent in the plasma at ime zero (Cpo) than was observed for the pharmaceutical agent in the free form. Also observed was a longer serum or plasma half-life (T 1/2) for the cephalosporin:lipid complex form than for the free form, and a greater area under a graphic plot of serum or plasma concentration vs time, termed area under curve (AUC).

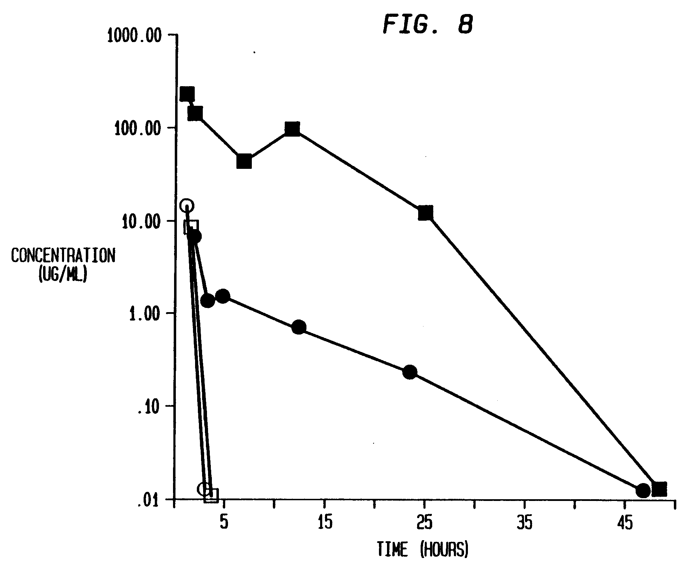

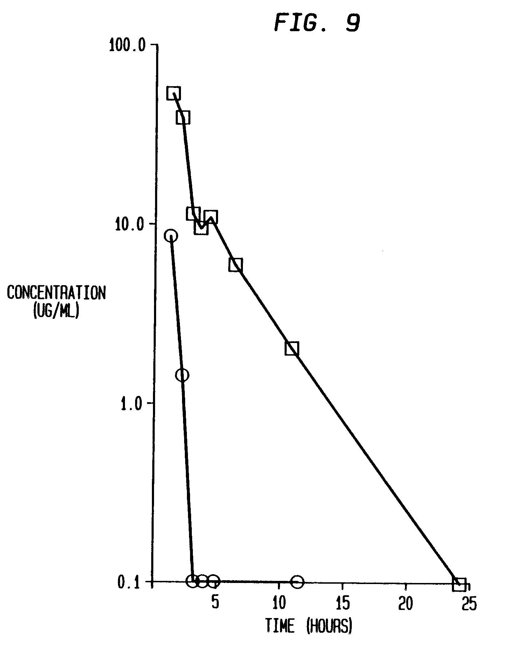

Figure 8 shows serum levels of cephapirin activity in mice (each point represents 3-5 mice) at intervals after injection of free cephapirin of cephalosporin:lipid complex cephapirin (each, 200mg/kg), while Figure 9 shows cefazolin activity (each, 100mg/kg) after a single intravenous dose of cefazolin administered in a conventional aqueous solution (circles) or in complex composed of DPPC with a final drug to lipid ratio of 1:5 by weight (squares). Both cephapirin and cefazolin display prolonged activity in serum and activity at a higher level by the cephalosporin:lipid complex preparation as compared to the free drug. It can be seen that in both Fig. 8 and Fig. 9, the cephapirin/cefazolin level and hence activity in the serum or plasma was greater and more prolonged with the cephalosporin:lipid dosage form when compared to the free drug. Extended prophylactic activity in the serum can also be achieved by subcutaneous administration of a cephalosporin:lipid complex antibiotic.

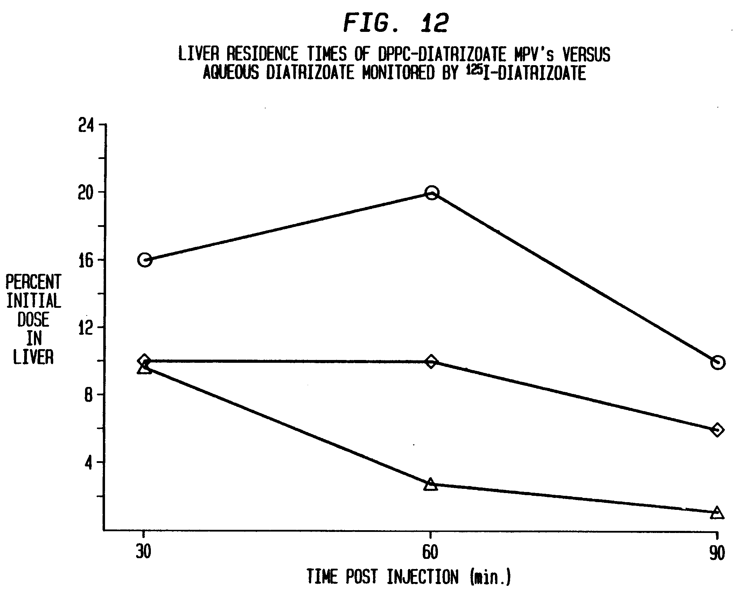

Contrast agents were tested in mice by injecting a single intravenous dose of free or complexed contrast agent (diatrizoate) via the tail vein. At a dose of 924mg/kg iodine the level of greater than about 2mgs I per gram of liver was achieved. (Fig. 12). As shown in Fig. 12 and Table 3, the final iodine/lipid ratio in the MPVs was 4.5:1 (w/w); the iodine concentration was 38.5 mg/ml and the lipid concentration was 8.5 mg/ml. The specific activity of the doses were about 10000 c/mg I.

The efficacy of prophylactic use of antibiotics may be tested by a number of methods well known to those skilled in the art. Several models for prophylactic efficacy testing are briefly described below.

Staphylococcal Abscess Model. S. aureus (ATCC #29740) was grown overnight at 30°C in trypticase soy broth. Aliquots of this suspension were frozen at -70°C. The in vitro minimal inhibitory concentration (MIC) of cephapirin sodium for this strain was shown to be 0.05-0.10ug/ml by the micro-titer dilution method. For use, cultures were thawed and diluted with saline to contain the desired number of colony forming units (CFU) per ml. Mice (out-bred CD-1 males) were injected subcutaneously with 0.1 ml of S. aureus suspension in the abdominal area. Treatment with free or encapsulated drug was given before or after infection. At four days after infection, mice were killed by cervical dislocation, the abscesses were carefully dissected out and their diameters measured.

Staphylococcal Septicemia Model. The S. aureus inoculum was prepared as for the abscess model. Mice were inoculated intraperitoneally with 2 x 10⁸ CFU in 5% hog/mucin. Treatment was given before or after infection. Mortality was monitored for 3 days post-infection.

Salmonella typhimurium Septicemia Model: S. typhimurium (ATCC #14028) was grown in trypticase soy broth at 35°C overnight. Aliquots of this suspension were frozen and maintained at -70°C. For use, cultures were thawed and diluted to contain 700 CFU/ml in 0.9% saline. Mice were inoculated intravenously (0.1 ml) via the lateral tail vein. Treatment was given at various times after infection. Mortality was monitored daily for two weeks.

In addition to providing prolonged serum antibacterial activity levels, the EPC liposome formulation targets the drug to particular organs when given intravenously. This particularly includes the reticulo-endothelial system and most particularly the spleen.

Salmonella typhimurium is a facultative intracellular bacterium capable of causing systemic infection in mice. Spontaneous mouse salmonellosis usually results from ingestion of the organism (via contaminated feed, bedding or feces). Infection can also occur by a conjunctival route or after experimental sub-cutaneous, intraperitoneal or intravenous inoculation. In all cases, the organism gains access to the systemic circulation causing a septicemia followed by localization with the major reticulo-endothelial organs (spleen, liver, lymph nodes). The bacteria can survive and multiply within macrophages and will cause a chronic infection unless death intervenes.

The high ratio cephalosporin complex administered to test animals substantially increased survival time at all doses and treatment times examined and exceeded free cephalosporin administration.

Cephalosporin:lipid complex cefazolin provides significant prophylactic activity, a Tₜ for periods of time that first exceed that of the free pharmaceutical agent. Clearly the high ratio cephalosporin complex was significantly longer acting than the free cephalosporin.

In studies of tissue levels of cephalosporin it has been found that tissue levels of cephalosporin following administration of high ratio cephalosporin complex may exceed plasma levels in both concentration of drug and length of time of drug retention. It is therapeutically effective in prophylaxis for periods of up to seven days or longer as the cephalosporin is bioavailable to stop bacterial proliferation. Therefore loading of an RES organ -- a concomitant of high ratio cephalosporin complex administration --such as the spleen with cephalosporin, even in the absence of detectable plasma levels of drug can afford protection against organisms in the blood (e.g., bacteremia)

The method of this invention is affected by complex size in some embodiments. Prophylactic uptake by the RES occurs most rapidly with complexes greater than 0.5u in diameter. Thus if extended term plasma levels of cephalosporin are the primary objective, this is promoted by using complexes of less than 0.5u in diameter while RES loading is facilitated by the use of complexes of about 0.5u or greater. In either situation the RES will ultimately be the repository of much of an administered dose of high ratio cephalosporin complex.

While conventional liposomes are useful for prophylaxis in the practice of this invention including MLVs, small sonicated unilamellar vesicles, large unilamellar vesicles, LUVETs, SPLVs, FATMLVs, steroidal liposomes or alpha-tocopherol based vesicles, the high ratio cephalosporin:lipid complexes are preferred.

In a lipid based drug delivery system, a bioactive agent such as a drug is entrapped in or associated with a lipid such as in a cephalosporin:lipid entity and then administered to the patient to be treated. For example, see Rahman et al., U.S. Patent No. 3,993,754; Sears, U.S. Patent No. 4,145,410; Papahadjopoulos et al., U.S. Patent No. 4,235,871; Schneider, U.S. Patent No. 4,114,179; Lenk et al., U.S. Patent No. 4,522,803; and Fountain et al., U.S. Patent No. 4,588,578.

The mode of administration of the active agent:lipid complex may determine the sites and cells in the organism to which the compound will be delivered. Cephalosporin:lipid complex (or other antibacterial or anti-infective agents) can be administered alone but will generally be administered in admixture with a pharmaceutical carrier selected with regard to the intended route of administration and standard pharmaceutical practice. Iodinated contrast agent complex will be similarly administrable. The preparations may be injected parenterally, for example, intra-arterially or intravenously. The preparations may also be administered via oral, subcutaneous, or intramuscular routes, or by inhalation. For parenteral administration, they can be used, for example, in the form of a sterile aqueous solution which may contain other solutes, for example, enough salts or glucose to make the solution isotonic. Other uses, depending upon the particular properties of the preparation, may be envisioned by those skilled in the art. In some embodiments high ratio cephalosporin:lipid complex cephalosporin may be employed in unit dosage form adapted to convenient administration to a subject animal including a human.

A "therapeutically effective amount" will be understood to mean a sufficient amount to achieve a physical or physiological response The therapeutically effective amount of a given cephalosporin will vary with the mode of the administration, the particularities of the recipient, the sensitivity of the target bacteria, and other factors well known in the art. A therapeutically effective amount of contrast agent will vary with the aspect of the subject to be imaged and with the nature of the X-ray imaging equipment but will be easily determinable by empirical observation by those skilled in the art.

For administration to humans in the curative treatment of disease states or for imaging, the prescribing physician will ultimately determine the appropriate dosage for a given human subject, and this can be expected to vary according to the age, weight, and response of the individual as well as the nature and severity of the patient's disease. The dosage of the drug in cephalosporin:lipid complex form or for iodinated contrast agent:lipid complex will generally be about that employed for the free active agent. In some cases, however, it may be necessary to administer doses outside these limits.

### EXAMPLE 1

### Preparation of High Ratio Cephalosporin:Lipid Complex

In a 50 ml round-bottom flask, 92 ul of a 300 mg/ml cefazolin sodium stock solution was diluted to 2 mls by adding 1.908 mls dH₂O (total 26.2 mgs of drug). 2 mls of 100% ethanol was added along with 2.5 mls of a 20 mg/ml lipid (DPPC) stock solution (total 50 mgs of lipid). The flask was immersed in a 37°C circulating water bath while a stream of air was used to agitate the resulting mixture. The stream of air was adjusted so that it was strong enough to thoroughly mix and stir the mixture during solvent removal. After 1.5 to 2 minutes the mixture changed from a dispersed biphase (cloudy) to a monophase (clear). As solvent removal continued the mixture became opaque and gelatinous or gel-like. The gel state indicated continued hydration. The purging was continued for 5 minutes at which time only a very faint odor of ethanol was detected. At this point the cephalosporin:lipid:aqueous mixture was thick and somewhat gelatinous. The mixture was briefly heated in a 51°C circulating water bath until the mixture liquified; this required about 15 seconds. The mixture was then transferred to 2-1.5ml conical microfuge tubes and spun at 15000 rpm in the microfuge for 10-15 minutes forming a cephalosporin:lipid complex pellet. The aqueous phase was removed from above the pellet and 145mM saline was added to fill the tube. The tube was vortexed briefly until the pellet was thoroughly resuspended. The cephalosporin:lipid complex was recentrifuged and resuspended as above. The resulting cephalosporin:lipid complex was washed two more times either as described above or may be combined with other materials including other batches of cephalosporin:lipid complex for subsequent washing. If the complexes are to be combined, each was transferred to a single Corex™ tube (Corning Glass Works, Corning, NY) after the first resuspension and spun at 10000g for 10-15 minutes. The supernatant was removed from the pellet and the pellet was resuspended in saline as described above. The washed pellet was assayed for drug, lipid and residual ethanol. The results of such tests disclosed 18.1mg drug/ml, 32.3mg/ml lipid and 2% residual alcohol.

### EXAMPLE 2

### Preparation of High Ratio Cephalosporin:Lipid Complex

In a 50 ml round-bottom flask, 217ul of a 300 mg/ml cefazolin sodium stock solution was diluted to 2 mls by adding 1.783 mls dH₂O (total 65.1 mgs of drug). 2 mls of 100% ethanol was added along with 2.5 mls of a 20 mg/ml lipid (DPPC) stock solution (total 50 mgs of lipid). The flask was immersed in a 37°C circulating water bath while a stream of air was used to agitate the resulting mixture. The stream of air was adjusted so that it was strong enough to thoroughly mix and stir the mixture during solvent removal. After 1.5 to 2 minutes the mixture changed from a dispersed biphase (cloudy) to a monophase (clear). As solvent removal continued the mixture became opaque and gelatinous or gel-like. The gel state indicated continued hydration. The purging was continued for 5 minutes at which time only a very faint odor of ethanol was detected. At this point the cephalosporin:lipid:aqueous mixture was thick and somewhat gelatinous. The mixture was briefly heated in a 51°C circulating water bath until the mixture liquifies; this required about 15 seconds. The mixture was then transferred to 2-1.5ml conical microfuge tubes and spun at 15000 rpm in the microfuge for 10-15 minutes forming a cephalosporin:lipid complex pellet. The aqueous phase was removed from above the pellet and 145mM saline was added to fill the tube. The tube was vortexed briefly until the pellet was thoroughly resuspended. The cephalosporin:lipid complex was recentrifuged and resuspended as above. The resulting cephalosporin:lipid complex was washed two more times either as described above or may be combined with other materials including other batches of cephalosporin:lipid complex for subsequent washing. If the complexes are to be combined, each was transferred to a single Corex™ tube (Corning Glass Works, Corning, NY) after the first resuspension and spun at 10000g for 10-15 minutes. The supernatant was removed from the pellet and the pellet was resuspended in saline as described above. The washed pellet was assayed for drug, lipid and residual ethanol. The results of such tests disclosed 11.5mg drug/ml, 14.9mg lipid/ml and 2% residual alcohol. The trapping efficiency of the cephalosporin was 74% and the final cephalosporin:lipid ratio was 0.77:1.

### EXAMPLE 3

### Preparation of High Ratio Cephalosporin:Lipid Complex

In a 50 ml round-bottom flask, 434ul of a 300 mg/ml cefazolin sodium stock solution was diluted to 2 mls by adding 1.566 mls dH₂O (total 130 mgs of drug). 2 mls of 100% ethanol was added along with 2.5 mls of a 20 mg/ml lipid (DPPC) stock solution (total 50 mgs of lipid). The flask was immersed in a 37°C circulating water bath while a stream of air was used to agitate the resulting mixture. The stream of air was adjusted so that it was strong enough to thoroughly mix and stir the mixture during solvent removal. After 1.5 to 2 minutes the mixture changed from a dispersed biphase (cloudy) to a monophase (clear). As solvent removal continued the mixture became opaque and gelatinous or gel-like. The gel state indicated continued hydration. The purging was continued for 5 minutes at which time only a very faint odor of ethanol was detected. At this point the cephalosporin:lipid:aqueous mixture was thick and somewhat gelatinous. The mixture was briefly heated in a 51°C circulating water bath until the mixture liquifies; this required about 15 seconds. The mixture was then transferred to 2-1.5ml conical microfuge tubes and spun at 15000 rpm in the microfuge for 10-15 minutes forming a cephalosporin:lipid complex pellet. The aqueous phase was removed from above the pellet and 145mM saline was added to fill the tube. The tube was vortexed briefly until the pellet was thoroughly resuspended. The cephalosporin:lipid complex was recentrifuged and resuspended as above. The resulting cephalosporin:lipid complex was washed two more times either as described above or may be combined with other materials including other batches of cephalosporin:lipid complex for subsequent washing. If the complexes are to be combined, each was transferred to a single Corex™ heavy duty glass tube (Corning Glass Works, Corning, NY) after the first resuspension and spun at 10000g for 10-15 minutes. The supernatant was removed from the pellet and the pellet was resuspended in saline as described above. The washed pellet was assayed for drug, lipid and residual ethanol. The results of such tests disclosed 19mg drug/ml, 13.9mg lipid/ml and 2% residual alcohol. The trapping efficiency of the cephalosporin was 60% and the final cephalosporin:lipid ratio was 1.43:1.

### Example 4

### Cephalosporin:Lipid -- Preparation

2.5ml of a 50mg/ml cephapirin sodium solution was used to disperse and hydrate 200mg CHSₜᵣᵢₛ with mechanical stirring. After stirring the resulting suspension for 1 minute, the suspension was placed on a rocker for 3 hours at 4°C. The suspension was uniform in appearance and was then diluted to 3 mls with phosphate buffered saline (Mg²⁺ and Ca²⁺ free) at pH 7.4. The final suspension contained 40mg cephapirin/ml as determined by agar diffusion assay.

### Example 5

### Cephalosporin:Lipid Complex -- Preparation

2g of CHSₜᵣᵢₛ and 1.075g of cephapirin sodium were weighed out and transferred to a 125ml Erlenmeyer flask. 20ml of distilled water was added to the flask and the mixture shaken over night at 4°C. 35ml phosphate buffered saline (Mg²⁺ and Ca²⁺ free) at pH7.4 was added to the mixture which was then transferred to 2 30ml Corex™ tubes and centrifuged at 10,000g for 30 minutes. The supernatant was decanted, and the pellet of cephalosporin:lipid complex was resuspended in 20ml phosphate buffered saline per tube, and the tubes recentrifuged as above. The final pellet volume was 22ml. The final drug concentration in the pellet was 17. 1mg/ml as measured by agar diffusion assay. The final cephalosporin:lipid ratio was 6:1.

### Example 6

### Cephalosporin:Lipid Complex -- Preparation

20ml of a stock solution of EPC at 100mg/ml in CHCl₃ was diluted to 200ml with 100% ethanol in a 500ml round-bottom flask. 1.075g of cephapirin sodium dissolved in distilled water sufficient for a final volume of 6ml was added. A clear yellow monophase resulted. The monophase was placed on a rotary evaporator with a 40°C water bath and pressure of 740mmHg until the monophase appeared as a dry lipid/drug film and no ethanol odor was detected. Rotary evaporation took 30 minutes ± 5. The film was resuspended in 30ml phosphate buffered saline and transferred to 2 30ml Corex™ tubes. The tubes were centrifuged at 10,000g for 10 minutes. The supernatants ware decanted, the pellets resuspended in 10ml phosphate buffered saline per tube and recentrifuged as above. The final pellets of cephalosporin:lipid complex was 12ml. The final drug concentration was 15.7mg/ml by agar diffusion assay and the final cephalosporin:lipid ratio was 11:1 (w/w).

### Example 7

### Cephalosporin:Lipid Complex -- Prolonged Serum Activity

Cephapirin activity in the serum of mice was determined at intervals after subcutaneous or intravenous administration of free or complexed drug. Figure 8 shows the results of a typical experiment. Maximal activity was found in the serum of mice given free cephapirin (by either route) at the earliest time points examined (one half to one hour post-injection). The half-life of free drug was estimated to be approximately 20 minutes for both intravenous and sub-cutaneous administration. For free drug no activity was detectable in the serum at 3 hours or later. Animals given similar doses of unwashed CHS-tris cephapirin (with approximately 30% of the administered dose encapsulated) subcutaneously showed prolonged serum activity with levels of approximately 0.2 ug/ml (0.01% of the original dose) still present at 24 hours post-injection. Mice that received washed EPC complex (where 100% of the administered dose was encapsulated) by the intravenous route also showed prolonged serum activity. With a 200 mg/kg dose, serum levels were 10 ug/ml or greater for 24 hours after injection. In both cephalosporin:lipid complex cases, the apparent half-life of the drug in serum was greater than four hours.

Intravenous administration of 200 mg/kg of free cephapirin resulted in detectable activity in the liver and kidney one hour after injection (Table 1). No activity was detected at 3 hours or later. Mice that received EPC cephalosporin:lipid complex showed activity in the kidney for 24 hours after injection. The spleen and liver showed greatly enhanced activity that persisted for at least 24 hours.

### Example 8

### Cephalosporin:Lipid Complex -- Prolonged Serum Activity

Intravenous injection of a single 100mg/kg dose of free cefazolin dosium in mice results in a mean serum concentration of 8 to 20ug/ml at one hour post dose (Fig. 9). The serum level declined rapidly to approximately 2ug/ml at 2 hours and is below the level of detection at 3 hours after administration. In contrast, animals that received the cephalosporin:lipid complex containing the same 100mg/kg dose of cefazolin showed serum concentrations of 55 to 80ug/ml at one hour and still had levels of 2 to 3ug/ml at 12 hours post dose.

### Example 9

### Cephalosporin:Lipid Complex

### Prophylaxis of Localized Staphylococcal Infection

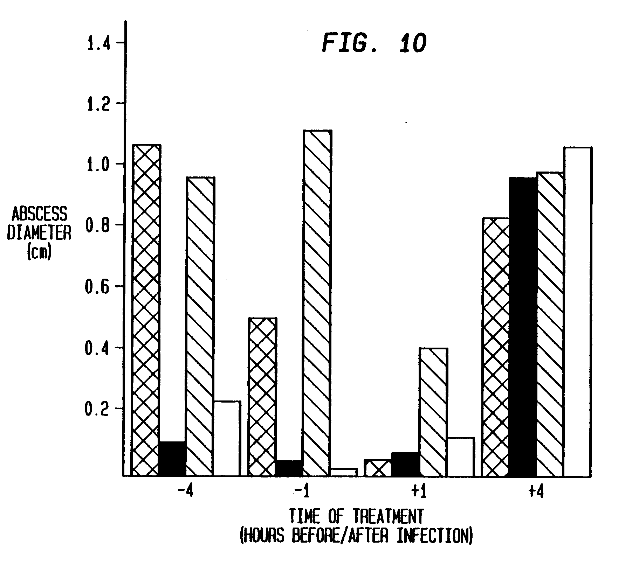

To determine the effect of treatment on abscess development, mice were given a single intravenous or subcutaneous injection of free or complexed cephapirin at various times before or after injection. Fig. 10 shows that treatment in a single dose (200 mg/kg) with free cephapirin (by either route) has no effect on abscess size when given at four hours before infection. Treatment at one hour before infection reduced abscess size slightly while treatment at one hour after infection significantly reduces or prevents abscess development. In contrast, treatment with cephalosporin:lipid complex cephapirin (either iv with EPC complex or sc with CHSt complex) affords significant protection against abscess development when administered up to four hours before infection as well as when given at one hour after infection. Additional investigation found that empty EPC liposomes given iv or empty CHSₜ liposomes given sc had no effect on abscess size, and empty EPC liposomes plus free drug gave results similar to free drug alone.

### Example 10

### Cephalosporin:Lipid Complex

### Prophylaxis of Systemic Staphylococcal Infection

The effect of free and CHSₜᵣᵢₛ cephalosporin:lipid complex cephapirin was compared in an acute staphylococcus septicemia model. Table 2 shows that 70 to 80% of untreated animals die within 72 hours of infection. Mice treated subcutaneously with free or cephalosporin:lipid complex at one hour before infection survived until the end of the experiment (14 days). Treatment at four hours before infection with cephalosporin:lipid complex protected the majority of mice whereas treatment with free drug at that time had no effect on survival.

### Example 11

### Cephalosporin:Lipid Complex

### Therapy of Systemic Salmonellosis

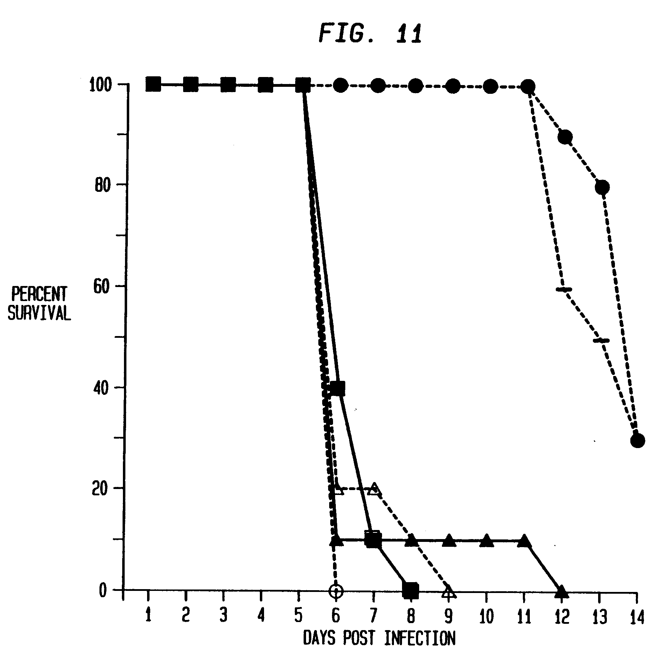

Mice (in groups of 10) that were inoculated intravenously with 700 CFU of S. typhimurium and received no treatment died within 6-8 days after infection (Fig. 11). Treatment on days 2 and 4 post-inoculation with free cephapirin by the subcutaneous or I.V. route did not prolong survival. Intravenous treatment with cephapirin in unwashed stearylamine containing EPC complex enhanced survival. In this case, the cephalosporin:lipid complex preparation was not washed so that 30% of the administered dose was encapsulated.

### Example 12

### Contrast Agent:Lipid Complex -- Preparation

Two mls diatrizoate (Renografin-76™, Squibb, Princeton, N.J.) in the form of sodium and meglumine salts at 370mg/ml was placed in a 100 ml round-bottom flask. To this was added 2 mls of absolute ethanol while mixing followed by addition of 50 mgs of hydrogenated soy phosphatidylcholine in 2.5 ml chloroform. The material in the flask was mixed by swirling the flask vigorously forming an emulsion. The chloroform organic solvent phase was removed by using a stream of nitrogen at a flow rate of between about 5-10Lpm to stir the material while the flask is immersed in a 30°C water bath. Solvent removal was continued for 10 minutes. Then 7 mls of 0.9% saline was added to the flask and mixing was accomplished by swirling. The material was then transferred to a 15ml Corex™ tube and centrifuged at approximately 10,000Xg for 15 minutes at 20°C. The supernatant was removed and the pellet resuspended in 10 mls 0.9% saline, and the washing procedure repeated two more times. The resulting contrast agent:lipid complex was found to contain 58.7 mg/ml iodine (94.7mg/ml diatrizoate) and 20.8 mg/ml phospholipid for a final iodine:lipid ratio of 2.8:1.

**TABLE 1**

| Tissue distribution of cephapirin activity after intravenous administration of free or EPC complex formulation | | | | | | |
|---|---|---|---|---|---|---|
| TISSUE | FORMULATION | 1 | 3 | 7 | 10 | 24 |
| KIDNEY | Free | 49 ± 8 | <0.5 | <0.5 | <0.5 | <0.5 |
| | EPC complex | 118 ± 15 | 43 ± 6 | 25 ± 3 | 15 ± 6 | 2 ± 1 |
| SPLEEN | Free | <0.5 | <0.5 | <0.5 | <0.5 | <0.5 |
| | EPC complex | 1202 ± 12 | 1243 ± 305 | 1031 ± 181 | 570 ± 95 | 67 ± 2 |
| LIVER | Free | 46 ± 12 | < 0.5 | < 0.5 | <0.5 | <0.5 |
| | EPC complex | 171 ± 12 | 228 ± 34 | 143 ± 48 | 149 ± 22 | 66 ± 22 |

| | | | | | | |
|---|---|---|---|---|---|---|
| a) Values given are the mean of 3 to 5 mice ± S.E.M. | | | | | | |

**TABLE 2**

| Effect of Free and CHS-tris complex Encapsulated Cephaprin on Survival of Mice with Acute Staphylococal Stepticemia | | | |
|---|---|---|---|
| TIME OF TREATMENT | DOSE (mg/kg) | % Survival at 72 hours^{a} | |
| | | Free Cephapirin | CHSt Cephapirin |
| -1 hour | 200 | 100 | 90 |
| | 100 | 100 | 100 |
| | 50 | 100 | 100 |
| -4 hour | 200 | 0 | 100 |
| | 100 | 10 | 80 |
| | 50 | 0 | 50 |
| NO TREATMENT | | 20 | |

| | | | |
|---|---|---|---|
| a) Mice were treated subcutaneously at one or four hours before intraperitoneal injection of 2 x 10⁸ CFU of S. aureus. There were 10 mice in eaeach group. | | | |

**TABLE 3**

| Effect of Free and CHS-tris complex Encapsulated Cephaprin on Survival of Rice with Acute Staphylococal Stepticemia | | | | | | |
|---|---|---|---|---|---|---|
| mgI Dosed | mg/kg Iodine | mg lipid Dosed | mg/kg Lipid | mg I/g Liver | | |
| | | | | 30' | 60' | 90' |
| 15.4 | 616 | 3.4 | 136 | 1.3 | 1.0 | 0.7 |
| 23.1 | 924 | 5.1 | 204 | 3.0 | 3.2 | 1.6 |
| 23.1 | 924 | --- | --- | 2.8 | 0.8 | 0.3 |

## Claims

1. A method of preparing high ratio active agent:lipid complex comprising a lipid and an active agent, the method comprising:
a. providing the active agent in a monophase comprising a first aqueous phase, aqueous miscible organic solvent, and lipid;
b. evaporating organic solvent from the monophase, while maintaining the active agent and lipid in hydrated form; and
c. adding a second aqueous phase to the evaporated monophase of step (b) to form liposomes.

2. The method of Claim 1 wherein the step of providing the active agent in a monophase comprises initially providing the active agent in a biphasic mixture, the biphasic mixture comprising a first aqueous phase, aqueous miscible organic solvent, aqueous immiscible organic solvent, and lipid; and evaporating the biphasic mixture to form said monophase.

3. The method of Claim 2 wherein said biphasic mixture comprises about equal amounts (v/v) of (i) the active agent dissolved in the first aqueous phase, (ii) said aqueous miscible organic solvent, and (iii) said lipid dissolved in said aqueous immiscible organic solvent, wherein said aqueous miscible organic solvent is ethanol and said aqueous immiscible organic solvent is chloroform.

4. The method of Claim 1, 2 or 3 wherein the lipid is a rigid lipid and the active agent is a cephalosporin, wherein the resultant cephalosporin:lipid complex is at least 20% (w/w) cephalosporin, and optionally wherein the evaporating is by positive pressure at 30 to 37°C.

5. The method of Claim 4 wherein the cephalosporin of the resultant cephalosporin:lipid complex is at least 30% (w/w), optionally at least 40% (w/w), and further optionally at least 50% (w/w).

6. The method of Claim 4 or 5 wherein the lipid comprises a head group and two carbon chains, said chains being saturated and of a length of at least 16 carbon units, optionally wherein the lipid is dipalmitoylphosphatidylcholine, and further optionally wherein said dipalmitoylphosphatidylcholine comprises up to 80% (w/w) of the complex.

7. The method of any one of Claims 4 to 6 wherein the cephalosporin is cefazolin, cephapirin, cephalothin, cefaclor, cephaloridine, cephoxazole, cefoxitin, cephradine, cephalexin, cephaloglycin, cefuroxime, cefmenoxime or cephalonium.

8. A high ratio cephalosporin:lipid complex comprising a lipid and a cephalosporin and optionally wherein said lipid is a rigid lipid, and wherein the cephalosporin:lipid complex is at least 20% (w/w) cephalosporin.

9. The complex of Claim 8 wherein the cephalosporin is at least 30% (w/w), optionally at least 40% (w/w), and further optionally at least 50% (w/w).

10. The complex of Claim 8 or 9 wherein the lipid comprises a head group and two carbon chains, said chains being saturated and of a length of at least 16 carbon units, optionally wherein the lipid is dipalmitoylphosphatidylcholine, and further optionally wherein said dipalmitoylphosphatidylcholine comprises up to 80% (w/w) of the complex.

11. The complex of any one of Claims 8 to 10 wherein the cephalosporin is cefazolin, cephapirin, cephalothin, cefaclor, cephaloridine, cephoxazole, cefoxitin, cephradine, cephalexin, cephaloglycin, cefuroxime, cefmenoxime or cephalonium.

12. A composition for use in bacterial infection prophylaxis in an animal, including a human, comprising an effective amount of cephalosporin:lipid complex of any one of Claims 8 to 11.

13. The composition of Claim 12 wherein the bacterial infection prophylaxis is against bacteremia ; a disseminated bacterial infection involving the reticulo-endothelial system, the bacteria Mycobacterium sp; or osteomyelitis.

14. The method of preparing high ratio active agent:lipid complex of Claim 1, 2, or 3 wherein the active agent is an iodinated contrast agent, wherein the iodine to lipid ratio of the complex is greater than 1:1 (w/w), and optionally wherein the evaporating is by positive pressure at 30 to 75°C.

15. The method of Claim 14 wherein the iodine to lipid ratio of the complex is at least 2:1 (w/w), optionally at least 4:1 (w/w), and further optionally at least 8:1 (w/w).

16. The method of Claim 14 or 15 wherein the lipid comprises a rigid lipid, optionally wherein the lipid comprises a head group and two carbon chains, said chains being saturated and of a length of at least 16 carbon units, and further optionally said lipid being dipalmitoylphosphatidylcholine, said dipalmitoylphosphatidylcholine optionally comprising as little as 20% to 7% (w/w) of the complex.

17. The method of Claim 14 or 15 wherein the lipid comprises a phosphatidylcholine.

18. The method of any one of Claims 14 to 17 wherein the contrast agent is diatrizoate, iocetamic acid, iodamide, iodoximate, iopamide, iopamidol, iopanoic acid, iophendylate, iothalamate, iothalamic acid, ipodate calcium, ipodate sodium, metrizamide or tyropanate or salts thereof.

19. A high ratio iodinated contrast agent:lipid complex comprising a lipid and an iodinated contrast agent wherein the iodine to lipid ratio of the complex is greater than 1:1 (w/w).

20. The complex of Claim 19 wherein said lipid is a rigid lipid, optionally wherein the lipid comprises a head group and two carbon chains, wherein said chains are saturated and of a length of at least 16 carbon units, and further optionally wherein said lipid is dipalmitoylphosphatidylcholine, said dipalmitoylphosphatidylcholine optionally comprising as little as 20 to 7% (w/w) of the complex.

21. The complex of Claim 19 wherein the lipid is a phosphatidylcholine.

22. The complex of any one of Claims 19 to 21 wherein the iodine to lipid ratio of the complex is at least 2:1 (w/w), optionally at least 4:1 (w/w), and further optionally at least 8:1 (w/w).

23. The complex of any one of Claims 19 to 22 wherein the contrast agent is diatrizoate, diatrizoic acid, iocetamic acid, iodamide, iodoximate, iohexol, iopamide, iopamidol, iopanoic acid, iophendylate, iothalamate, iothalamic acid, ipodate, metrizamide or tyropanate or salts thereof.

24. A composition comprising an iodinated contrast agent: lipid complex for use in increasing X-ray density in an animal, including a human, comprising an effective amount of high ratio active agent:lipid complex of Claims 19 to 23.

## Patentansprüche

1. Verfahren zur Herstellung eines Hochverhältnis-aktives Mittel:Lipid-Komplexes, der ein Lipid und ein aktives Mittel umfaßt, wobei das Verfahren umfaßt:
a. Bereitstellen des aktiven Mittels in einer Monophase, die eine erste wässrige Phase, ein Wasser-mischbares organisches Lösungsmittel und ein Lipid umfaßt;
b. Abdampfen des organischen Lösungsmittels aus der Monophase, während das aktive Mittel und das Lipid in hydratisierter Form erhalten wird; und
c. Zugeben einer zweiten wässrigen Phase zu der eingedampften Monophase von Schritt (b), um Liposome zu bilden.

2. Verfahren nach Anspruch 1, worin der Schritt des Bereitstellens des aktiven Mittels in einer Monophase umfaßt: Anfängliches Bereitstellen des aktiven Mittels in einer biphasischen Mischung, wobei die biphasische Mischung eine erste wässrige Phase, ein Wasser-mischbares organisches Lösungsmittel, ein nicht Wasser-mischbares organisches Lösungsmittel und ein Lipid umfaßt; und Eindampfen der biphasischen Mischung, um die genannte Monophase zu bilden.

3. Verfahren nach Anspruch 2, worin die genannte biphasische Mischung ungefähr gleiche Mengen (v/v) des (i) in der ersten wässrigen Phase gelösten aktiven Mittels, (ii) des genannten Wasser-mischbaren organisches Lösungsmittels und (iii) des im genannten nicht Wasser-mischbaren organischen Lösungsmittel gelösten Lipids umfaßt, worin genanntes Wasser-mischbares organisches Lösungmittel Ethanol ist, und genanntes nicht Wasser-mischbares organisches Lösungsmittel Chloroform ist.

4. Verfahren nach Anspruch 1, 2 oder 3, worin das Lipid ein rigides Lipid und das aktive Mittel ein Cephalosporin ist, worin der resultierende Cephalosporin:Lipid-Komplex mindestens 20% (w/w) Cephalosporin ist, und worin wahlweise das Abdampfen bei Überdruck bei 30 bis 37°C stattfindet.

5. Verfahren nach Anspruch 4, worin das Cephalosporin im resultierenden Cephalosporin:Lipid-Komplex mindestens 30% (w/w), wahlweise mindestens 40% (w/w) und weiter wahlweise mindestens 50% (w/w) beträgt.

6. Verfahren nach Anspruch 4 oder 5, worin das Lipid eine Kopfgruppe und zwei Kohlenstoffketten umfaßt, wobei die Ketten gesättigt und von einer Länge von wenigstens 16 Kohlenstoffeinheiten sind, worin das Lipid wahlweise Dipalmitoylphosphatidylcholin ist, und worin weiter genanntes Dipalmitoylphosphatidylcholin wahlweise bis zu 80% (w/w) des Komplexes umfaßt.

7. Verfahren nach irgendeinem der Ansprüche 4 bis 6, worin das Cephalosporin Cefazolin, Cephapirin, Cephalotin, Cefaclor, Cephaloridin, Cephoxazol, Cefoxitin, Cephradin, Cephalexin, Cephaloglycin, Cefuroxim, Cefmenoxim oder Cephalonium ist.

8. Hochverhältnis-Cephalosporin:Lipid-Komplex, der ein Lipid und ein Cephalosporin umfaßt, und worin wahlweise das genannte Lipid eine rigides Lipid ist, und worin der Cephalosporin:Lipid-Komplex wenigstens 20% (w/w) Cephalosporin ist.

9. Komplex nach Anspruch 8, worin das Cephalosporin wenigstens 30% (w/w), wahlweise wenigstens 40% (w/w) und weiter wahlweise wenigstens 50% (w/w) ist.

10. Komplex nach Anspruch 8 oder 9, worin das Lipid eine Kopfgruppe und zwei Kohlenstoffketten umfaßt, wobei genannte Ketten gesättigt und von einer Länge von wenigstens 16 Kohlenstoffeinheiten sind, worin das Lipid wahlweise Dipalmitoylphosphatidylcholin ist, und worin weiter genanntes Dipalmitoylphosphatidylcholin wahlweise bis zu 80% (w/w) des Komplexes umfaßt.

11. Verfahren nach irgendeinem der Ansprüche 8 bis 10, worin das Cephalosporin Cefazolin, Cephapirin, Cephalothin, Cefaclor, Cephaloridin, Cephoxazol, Cefoxitin, Cephradin, Cephalexin, Cephaloglycin, Cefuroxim, Cefmenoxim oder Cephalonium ist.

12. Zusammensetzung zur Verwendung in der Prophylaxe bakterieller Infektionen in einem Tier einschließlich Menschen, die eine wirksame Menge des Cephalosporin:Lipid-Komplexes nach irgendeinem der Ansprüche 8 bis 11 umfaßt.

13. Zusammensetzung nach Anspruch 12, worin die bakterielle Infektionsprophylaxe gegen Bakteriämie; eine disseminierte bakterielle Infektion, die das retikuloendotheliale System, die Bakterie Mycobacterium sp beinhaltet; oder Osteomyelitis ist.

14. Verfahren zur Herstellung eines Hochverhältnis-aktives Mittel:Lipid-Komplexes nach Anspruch 1, 2 oder 3, worin das aktive Mittel ein iodiertes Kontrastmittel ist, worin das Iod zu Lipid-Verhältnis des Komplexes größer als 1:1 (w/w) ist, und worin das Eindampfen wahlweise bei Überdruck bei 30 bis 75°C erfolgt.

15. Verfahren nach Anspruch 14, worin das Iod zu Lipid-Verhältnis des Komplexes mindestens 2:1 (w/w), wahlweise mindestens 4:1 (w/w) und weiter wahlweise mindestens 8:1 (w/w) beträgt.

16. Verfahren nach Anspruch 14 oder 15, worin das Lipid ein rigides Lipid umfaßt, worin das Lipid wahlweise eine Kopfgruppe und zwei Kohlenstoffketten umfaßt, wobei die genannten Ketten gesättigt und von einer Länge von mindestens 16 Kohlenstoffeinheiten sind, und weiter worin genanntes Lipid wahlweise Dipalmitoylphosphatidylcholin ist, genanntes Dipalmitoylphosphatidylcholin wahlweise so wenig wie 20% bis 7 % (w/w) des Komplexes umfaßt.

17. Verfahren nach Anspruch 14 oder 15, worin das Lipid Phosphatidylcholin umfaßt.

18. Verfahren nach irgendeinem der Ansprüche 14 bis 17, worin das Kontrastmittel Amidotrizoat, Iocetaminsäure, Iodamid, Iodoxamat (Iodoximat), Iopamid, Iopamidol, Iopansäure, Iofendylat, Iothalamat, Iotalaminsäure, Calciumiopodat, Natriumiopodat, Metrizamid oder Tyropanoat (Tyropanat) oder Salze davon ist.

19. Hochverhältnis-iodiertes Kontrastmittel:Lipid-Komplex, der ein Lipid und ein iodiertes Kontrastmittel umfaßt, worin das Iod zu Lipid-Verhältnis größer als 1:1 (w/w) ist.

20. Komplex nach Anspruch 19. worin genanntes Lipid ein rigides Lipid ist, worin das Lipid wahlweise eine Kopfgruppe und zwei Kohlenstoffketten umfaßt, worin die genannten Ketten gesättigt und von einer Länge von wenigstens 16 Kohlenstoffeinheiten sind, und worin weiter genanntes Lipid wahlweise Dipalmitoylphosphatidylcholin ist, wobei genanntes Dipalmitoylphosphatidylcholin wahlweise so wenig wie 20 bis 7% (w/w) des Komplexes umfaßt.

21. Komplex nach Anspruch 19, worin das Lipid Phosphatidylcholin ist.

22. Komplex nach irgendeinem der Ansprüche 19 bis 21, worin das Iod zu Lipid-Verhältnis des Komplexes mindestens 2:1 (w/w), wahlweise mindestens 4:1 (w/w), und weiter wahlweise mindestens 8:1 (w/w) beträgt.

23. Komplex nach jedem der Ansprüche 19 bis 22, worin das Kontrastmittel Amidotrizoat, Amidotrizoesäure, Iocetaminsäure, Iodamid, Iodoxamat (Iodoximat), Iohexol, Iopamid, Iopamidol, Iopansäure, Iofendylat, Iothalamat, Iotalaminsäure, Iopodat, Metrizamid oder Tyropanoat (Tyropanat) oder Salze davon ist.

24. Zusammensetzung, die einen iodiertes Kontrastmittel: Lipid-Komplex umfaßt, zur Verwendung zur Steigerung der Röntgenstrahlen-Dichte in einem Tier einschließlich Menschen, der eine wirksame Menge des Hochverhältnis-aktives Mittel:Lipid-Komplexes nach den Ansprüchen 19 bis 23 umfaßt.

## Revendications

1. Procédé de préparation d'un complexe agent actif:lipide à rapport élevé comprenant un lipide et un agent actif, dans lequel:
a. on fournit l'agent actif dans une monophase comprenant une première phase aqueuse, un solvant organique miscible aqueux, et un lipide;
b. on fait évaporer le solvant organique de la monophase, tout en maintenant l'agent actif et le lipide sous forme hydratée; et
c. on ajoute une seconde phase aqueuse à la monophase évaporée de l'étape (b) pour former des liposomes.

2. Procédé de la revendication 1 dans lequel l'étape de fourniture de l'agent actif dans une monophase comprend la fourniture initiale de l'agent actif dans un mélange biphasique, le mélange biphasique comprenant une première phase aqueuse, un solvant organique miscible aqueux, un solvant organique non-miscible avec une phase aqueuse, et un lipide; et l'évaporation du mélange biphasique pour former ladite monophase.

3. Procédé de la revendication 2 dans lequel ledit mélange biphasique comprend des quantités approximativement égales (v/v) de (i) l'agent actif dissous dans la première phase aqueuse, (ii) ledit solvant organique miscible aqueux, et (iii) ledit lipide dissous dans ledit solvant organique non miscible avec une phase aqueuse, où ledit solvant organique miscible aqueux est l'éthanol et ledit solvant organique non miscible avec une phase aqueuse est le chloroforme.

4. Procédé de la revendication 1, 2 ou 3 dans lequel le lipide est un lipide rigide et l'agent actif est une céphalosporine, où le complexe céphalosporine:lipide résultant est à au moins 20% (p/p) de céphalosporine, et facultativement où l'évaporation se fait par pression positive entre 30 et 37°C.

5. Procédé de la revendication 4 dans lequel la céphalosporine du complexe céphalospotine:lipide résultant est à au moins 30% (p/p), facultativement à au moins 40% (p/p), ou encore facultativement à au moins 50% (v/v).

6. Procédé de la revendication 4 ou 5 dans lequel le lipide comprend un groupe de tête et deux chaînes carbonées, lesdites chaînes étant saturées et d'une longueur d'au moins 16 unités carbonées, facultativement où le lipide est la dipalmitoylphosphatidylcholine, et en outre facultativement où ladite dipalmitoylphosphatidylcholine constitue jusqu'à 80% (p/p) du complexe.

7. Procédé de l'une quelconque des revendications 4 à 6 dans lequel la céphalosporine est la céfazoline, la céphapirine, la céphalothine, le céfaclor, la céphaloridine, le céphoxazole, la céphoxitine, la céphradine, la céphalexine, la céphaloglycine, la céfuroxime, la cefménoxime ou le céphalonium.

8. Complexe céphalosporine:lipide à rapport élevé comprenant un lipide et une céphalosporine et facultativement où ledit lipide est un lipide rigide, et où le complexe céphalosporine:lipide est à au moins 20% (p/p) de céphalosporine.

9. Complexe de la revendication 8 dans lequel la céphalosporine est à au moins 30% (p/p), facultativement à au moins 40% (p/p), ou encore facultativement à au moins 50% (p/p).

10. Complexe de la revendication 8 ou 9 dans lequel le lipide comprend un groupe de tête et au moins deux chaînes carbonées, lesdites chaînes étant saturées et d'une longueur d'au moins 16 unités carbonées, facultativement où le lipide est la dipalmitoylphosphatidylcholine, et en outre facultativement où ladite dipalmitoylphosphatidylcholine constitue jusqu'à 80% (p/p) du complexe.

11. Complexe de l'une quelconque des revendications 8 à 10 dans lequel la céphalosporine est la céfazoline, la céphapirine, la céphalothine, le céfaclor, la céphaloridine, le céphoxazole, la céfoxitine, la céphradine, la céphalexine, la céphaloglycine, la céfuroxime, la cefménoxime ou le céphalonium.

12. Composition pour application dans la prophylaxie d'une infection bactérienne chez un animal, y compris l'homme, comprenant une quantité efficace de complexe céphalosporine:lipide de l'une quelconque des revendications 8 à 11.

13. Composition de la revendication 12 dans laquelle la prophylaxie de l'infection bactérienne est dirigée contre la bactérémie; une infection bactérienne disséminée impliquant le système réticulo-endothélial, les bactéries Mycobacterium sp; ou l'ostéomyélite.

14. Procédé de préparation d'un complexe agent actif:lipide à rapport élevé de la revendication 1, 2 ou 3 dans lequel l'agent actif est un agent de contraste iodé, où le rapport de l'iode au lipide du complexe est supérieur à 1:1 (p/p), et facultativement où l'évaporation se fait par pression positive entre 30 et 75°C.

15. Procédé de la revendication 14 dans lequel le rapport de l'iode au lipide du complexe est d'au moins 2:1 (p/p), facultativement d'au moins 4:1 (p/p), ou encore facultativement d'au moins 8:1 (p/p).

16. Procédé de la revendication 14 ou 15 dans lequel le lipide comprend un lipide rigide, facultativement où le lipide comprend un groupe de tête et deux chaînes carbonées, lesdites chaînes étant saturées et d'une longueur d'au moins 16 unités carbonées, et en outre facultativement ledit lipide étant la dipalmitoylphosphatidylcholine, ladite dipalmitoylphosphatidylcholine constituant facultativement à peine 20% à 7% (p/p) du complexe.

17. Procédé de la revendication 14 ou 15 dans lequel le lipide comprend une phosphatidylcholine.

18. Procédé de l'une quelconque des revendications 14 à 17 dans lequel l'agent de contraste est le diatrizoate, l'acide iocétamique, l'iodamide, l'iodoximate, l'iopamide, l'iopamidol, l'acide iopanoïque, l'iophendylate, l'iothalamate, l'acide iothalamique, l'ipodate calcique, l'ipodate sodique, le métrizamide ou le tyropanate ou leurs sels.

19. Complexe agent de contraste iodé:lipide à rapport élevé comprenant un lipide et un agent de contraste iodé où le rapport de l'iode au lipide du complexe est supérieur à 1:1 (p/p).

20. Complexe de la revendication 19 dans lequel ledit lipide est un lipide rigide, facultativement où le lipide comprend un groupe de tête et deux chaînes carbonées, où lesdites chaînes sont saturées et d'une longueur d'au moins 16 unités carbonées, et en outre facultativement où ledit lipide est la dipalmitoylphosphatidylcholine, ladite dipalmitoylphosphatidylcholine constituant facultativement à peine 20 à 7% (p/p) du complexe.

21. Complexe de la revendication 19 dans lequel le lipide est une phosphatidylcholine.

22. Complexe de l'une quelconque des revendications 19 à 21 dans lequel le rapport de l'iode au lipide du complexe est d'au moins 2:1 (p/p), facultativement d'au moins 4:1 (p/p), ou encore facultativement d'au moins 8:1 (p/p).

23. Complexe de l'une quelconque des revendications 19 à 22 dans lequel l'agent de contraste est le diatrizoate, l'acide diatrizoïque, l'acide iocétamique, l'iodamide, l'iodoximate, l'iohexol, l'iopamide, l'iopamidol, l'acide iopanoïque, l'iophendylate, l'iothalamate, l'acide iothalamique, l'ipodate, le métrizamide ou le tyropanate ou leurs sels.

24. Composition comprenant un complexe agent de contraste iodé:lipide pour application afin d'accroître la densité des rayons X chez un animal, y compris un homme, comprenant une quantité efficace d'un complexe agent actif:lipide à rapport élevé des revendications 19 à 23.
